(19) Europäisches Patentamt
European Patent Office
Office européen des brevets

(11) Veröffentlichungsnummer: **0 256 367 B1**

(12) **EUROPÄISCHE PATENTSCHRIFT**

(45) Veröffentlichungstag der Patentschrift: **29.08.90**

(21) Anmeldenummer: **87110846.0**

(22) Anmeldetag: **27.07.87**

(51) Int. Cl.[5]: **C07D 295/08,** C07D 211/14, C07D 265/30, C07C 215/22, C07C 215/20, C07C 213/00, A01N 43/34, A01N 43/84, A01N 33/10, A01N 39/00

(54) **Substituierte Hydroxypropylamin-Derivate.**

(30) Priorität: **09.08.86 DE 3627071**

(43) Veröffentlichungstag der Anmeldung: **24.02.88 Patentblatt 88/8**

(45) Bekanntmachung des Hinweises auf die Patenterteilung: **29.08.90 Patentblatt 90/35**

(84) Benannte Vertragsstaaten: **AT BE CH DE ES FR GB IT LI NL SE**

(56) Entgegenhaltungen:
**EP-A- 0 000 333**
**EP-A- 0 129 321**
**GB-A- 1 591 267**
**GB-A- 2 158 823**

**CHEMICAL ABSTRACTS SERVICE, "Registry Handbook", 1965-1971 Seite 12453R**
**CHEMICAL ABSTRACTS, Band 57, Nr. 10, 12. November 1962, Columbus, Ohio, USA DR. KARL THOMAE GMBH "Basic sustituted carbinols" Spalte 12381, Zusammenfassung 12381i**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Weissmüller, Joachim, Dr., Carl-Langhans-Strasse 53, D-4019 Monheim(DE)**
Erfinder: **Krämer, Wolfgang, Dr., Rosenkranz 25, D-5093 Burscheid 2(DE)**
Erfinder: **Berg, Dieter, Dr., Gellertweg 27, D-5600 Wuppertal 1(DE)**
Erfinder: **Reinecke, Paul, Dr., Steinstrasse 8, D-5090 Leverkusen 3(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 256 367 B1

**Beschreibung**

Die vorliegende Erfindung betrifft neue substituierte Hydroxypropylamin-Derivate, mehrere Verfahren zu ihrer Herstellung und ihre Verwendung als Fungizide.

Es ist bereits bekannt, daß organische Schwefelverbindungen, wie beispielsweise das Zinkethylen-1,2-bisdithiocarbamat, gute fungizide Eigenschaften besitzen (vgl. R. Wegler, Chemie der Pflanzenschutz- und Schädlingsbekämpfungsmittel, Springer Verlag, 1970, Band 2, Seite 65ff).

Weiterhin ist bekannt, daß bestimmte 4-Phenyl-2-hydroxy-butyl-amino-Derivate fungizide Eigenschaften besitzen (vgl. EP-A 0 129 321 und GB-A 2 158 823).

Die Wirkung dieser Verbindungen ist jedoch, insbesondere bei niedrigen Aufwandmengen und -konzentrationen, in einigen Anwendungsbereichen nicht immer voll befriedigend.

Es wurden neue substituierte Hydroxypropylamin-Derivate der allgemeinen Formel (I) gefunden.

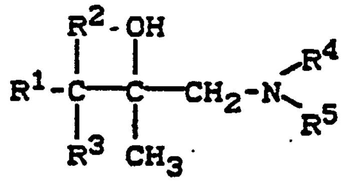 (I)

in welcher

$R^1$ für jeweils gegebenenfalls einfach oder mehrfach, gleich oder verschieden substituiertes Phenyl, Phenoxy, Phenylthio, Phenylalkyl, Phenoxyalkyl und Phenylthioalkyl mit jeweils 1 oder 2 Kohlenstoffatomen im Alkylteil, wobei als Phenylsubstituenten jeweils infrage kommen: Halogen; geradkettiges oder verzweigtes Alkyl, Alkoxy oder Alkylthio mit jeweils bis zu 5 Kohlenstoffatomen; Halogenalkyl, Halogenalkoxy oder Halogenalkylthio mit jeweils 1 oder 2 Kohlenstoffatomen und 1 bis 5 gleichen oder verschiedenen Halogenatomen, wie vorzugsweise Fluor- oder Chloratome; sowie für jeweils gegebenenfalls einfach oder mehrfach, gleich oder verschieden substituiertes Cyclohexyl, Cyclohexyloxy, Cyclohexylthio, Cyclohexylalkyl, Cyclohexyloxyalkyl und Cyclohexylthioalkyl mit jeweils gegebenenfalls 1 oder 2 Kohlenstoffatomen im Alkylteil, wobei als Cyclohexylsubstituenten jeweils infrage kommen: geradkettiges oder verzweigtes Alkyl und Alkoxy mit jeweils bis zu 5 Kohlenstoffatomen, Trifluormethyl oder Trifluormethoxy;

$R^2$ für Wasserstoff oder Methyl;

$R^3$ für Methyl oder Ethyl und

$R^4$ und $R^5$ unabhängig voneinander für geradkettiges oder verzweigtes Alkyl mit 1 bis 8 Kohlenstoffatomen oder für geradkettiges oder verzweigtes Alkenyl mit 3–6 Kohlenstoffatomen oder

$R^4$ und $R^5$ gemeinsam mit dem Stickstoffatom, an das sie gebunden sind, für einen gegebenenfalls einfach oder mehrfach, gleich oder verschieden substituierten 5- bis 7-gliedrigen, gesättigten Heterocyclus mit 1 oder 2 Heteroatomen, vorzugsweise Stickstoff und Sauerstoff stehen, wobei als Substituenten infrage kommen: Methyl, Ethyl und Hydroxymethyl, sowie deren pflanzenverträgliche Säureadditionsverbindungen und Metallsalzkomplexe, ausgenommen die Verbindung 1-N,N-Dimethylamino-2,3-dimethyl-4-(4-chlorophenyl)-2-butanol.

Die Verbindungen der Formel (I) können als geometrische und/oder optische Isomere oder Isomerengemische unterschiedlicher Zusammensetzung anfallen. Sowohl die reinen Isomeren als auch die Isomerengemische werden erfindungsgemäß beansprucht.

Weiterhin wurde gefunden, daß man

a) die neuen substituierten Hydroxypropylamin-Derivate der allgemeinen Formel (I)

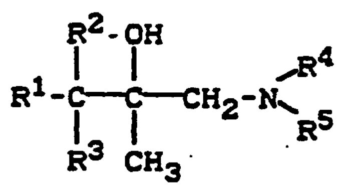 (I)

in welcher

$R^1$, $R^2$, $R^3$, $R^4$ und $R^5$ die oben angegebene Bedeutung haben,

ausgenommen die Verbindung 1-N,N-Dimethylamino-2, 3-dimethyl-4-(4-chlorophenyl)-2-butanol,

sowie deren pflanzenverträgliche Säureadditionsverbindungen und Metallsalzkomplexe erhält, wenn man Epoxide der allgemeinen Formel (II)

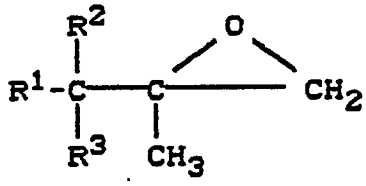

(II)

in welcher
$R^1$, $R^2$ und $R^3$ die oben angegebene Bedeutung haben, mit Aminen der allgemeinen Formel (III)

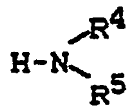

(III)

in welcher $R^4$ und $R^5$ die oben angegebene Bedeutung haben, gegebenenfalls in Gegenwart eines Verdünnungsmittels und gegebenenfalls in Gegenwart eines Katalysators umsetzt und gegebenenfalls anschließend eine Säure oder ein Metallsalz addiert; oder

b) die neuen substituierten Hydroxypropylamin-Derivate der allgemeinen Formel (Ia)

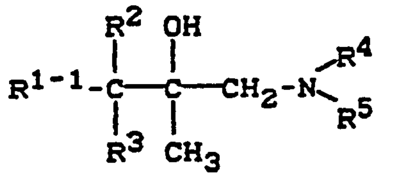

(Ia)

in welcher
$R^{1-1}$ für wie oben definiertes Cyclohexyl, Cyclohexyloxy, Cyclohexylalkyl oder Cyclohexyloxyalkyl steht und
$R^2$, $R^3$, $R^4$ und $R^5$ die oben angebene Bedeutung haben,
sowie deren pflanzenverträgliche Säureadditionsverbindungen und Metallsalzkomplexe erhält, wenn man entsprechende Hydroxypropylamin-Derivate der allgemeinen Formel (Ib)

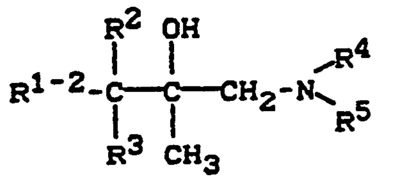

(Ib)

in welcher
$R^{1-2}$ für jeweils gegebenenfalls wie oben angegeben substituiertes Phenyl, Phenylalkyl, Phenoxyalkyl oder Phenoxy steht und $R^2$, $R^3$, $R^4$ und $R^5$ die oben angegebene Bedeutung haben,
gegebenenfalls in Gegenwart eines Verdünnungsmittels und gegebenenfalls in Gegenwart eines Katalysators sowie gegebenenfalls unter erhöhtem Druck hydriert; und gegebenenfalls anschliessend eine Säure oder ein Metallsalz addiert.

Weiterhin wurde gefunden, daß die neuen substituierten Hydroxypropylamin-Derivate der allgemeinen Formel (I) gute fungizide Eigenschaften besitzen. Dabei zeigen die erfindungsgemäßen Verbindungen der Formel (I) überraschenderweise eine höhere fungizide Wirksamkeit als das aus dem Stand der Technik bekannte Zinkethylen-1,2-bis-dithiocarbamat, welches wirkungsmäßig eine naheliegende Verbindung ist. Die erfindungsgemäßen Verbindungen stellen somit eine Bereicherung der Technik dar.

Die erfindungsgemäßen substituierten Hydroxypropylamin-Derivate sind durch die Formel (I) allgemein definiert.

Besonders bevorzugt sind Verbindungen der allgemeinen Formel (I), bei denen
$R^1$ für jeweils gegebenenfalls einfach bis dreifach, gleich oder verschieden substituiertes Phenylmethyl, Phenoxymethyl, Phenylthiomethyl, Phenoxy oder Phenylthio steht, wobei als Phenylsubstituenten jeweils infrage kommen: Fluor, Chlor, Brom, Methyl, Ethyl, Propyl, i-Propyl; n-, i-, s- und t-Butyl, n-Pentyl, 2-Methyl-2-butyl, Methoxy, Ethoxy, n- und i-Propoxy, t-Butoxy, Trifluormethyl, Trifluormethoxy und Trifluormethylthio; sowie für jeweils gegebenenfalls einfach bis dreifach, gleich oder verschieden substituiertes Cyclohexylmethyl, Cyclohexyloxymethyl, Cyclohexylthiomethyl, Cyclohexyloxy oder Cyclohexylthio steht, wobei als Cyclohexylsubstituenten jeweils infrage kommen: Methyl, Ethyl, n- und i-Propyl; n-, i-, s- und t-Butyl, n-Pentyl, 2-Methyl-2-butyl, Methoxy, Ethoxy, n- und i-Propoxy, t-Butoxy, Trifluormethyl und Trifluormethoxy;
$R^2$ für Wasserstoff oder Methyl steht;
$R^3$ für Methyl oder Ethyl steht und
$R^4$ und $R^5$ unabhängig voneinander für Methyl, Ethyl, n- und i-Propyl; n-, i-, s- und t-Butyl, n-Pentyl, Allyl, 2-Butenyl oder 3-Methyl-2-butenyl stehen, oder
$R^4$ und $R^5$ gemeinsam mit dem Stickstoffatom, an das sie gebunden sind, für jeweils gegebenenfalls einfach bis dreifach, gleich oder verschieden durch Methyl, Ethyl oder Hydroxymethyl substituiertes 1-Pyrrolidinyl, 1-Piperidinyl, 1-Piperazinyl, 4-Morpholinyl oder 1-Hexahydroazepinyl stehen.

Im einzelnen seien außer den bei den Herstellungsbeispielen genannten Verbindungen die folgenden Verbindungen der allgemeinen Formel (I) genannt:

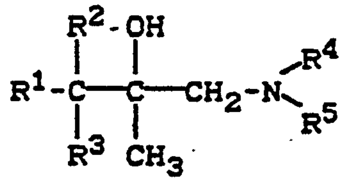

(I)

Tabelle 1

| $R^1$ | $R^2$ | $R^3$ | 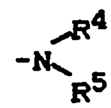 |
|---|---|---|---|
| 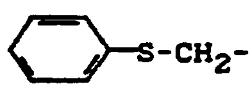 | $CH_3$ | $CH_3$ | 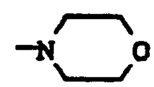 |
| 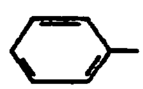 | $CH_3$ | $CH_3$ | 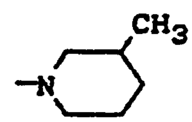 |
| 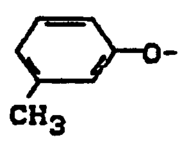 | $CH_3$ | $CH_3$ | 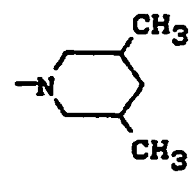 |

Tabelle 1 (Fortsetzung)

| $R^1$ | $R^2$ | $R^3$ | $-N{<}^{R^4}_{R^5}$ |
|---|---|---|---|
| 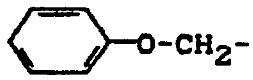 | $CH_3$ | $CH_3$ | 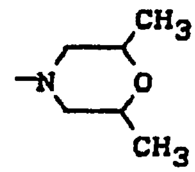 |
| 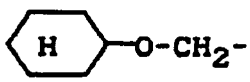 | $CH_3$ | $CH_3$ | 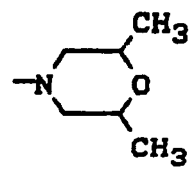 |
| 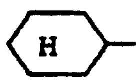 | $CH_3$ | $CH_3$ | 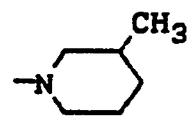 |
| 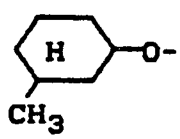 | $CH_3$ | $CH_3$ | 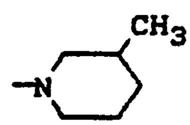 |

Verwendet man beispielsweise 4-(3-Chlorphenyl)-3,3-dimethyl-2-methyl-buten-1-oxid und Piperidin als Ausgangsstoffe, so kann der Reaktionsablauf des erfindungsgemäßen Verfahrens (a) durch das folgende Formelschema wiedergegeben werden:

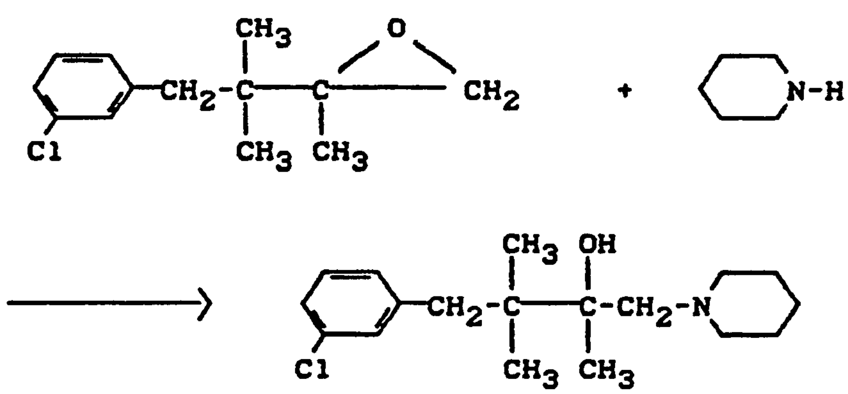

Verwendet man beispielsweise 3,3-Dimethyl-2-methyl-4-(3-methylphenyl)-1-(morpholin-4-yl)-2-butanol als Ausgangsstoff, so kann der Reaktionsablauf des erfindungsgemäßen Verfahrens (b) durch das folgende Formelschema wiedergegeben werden:

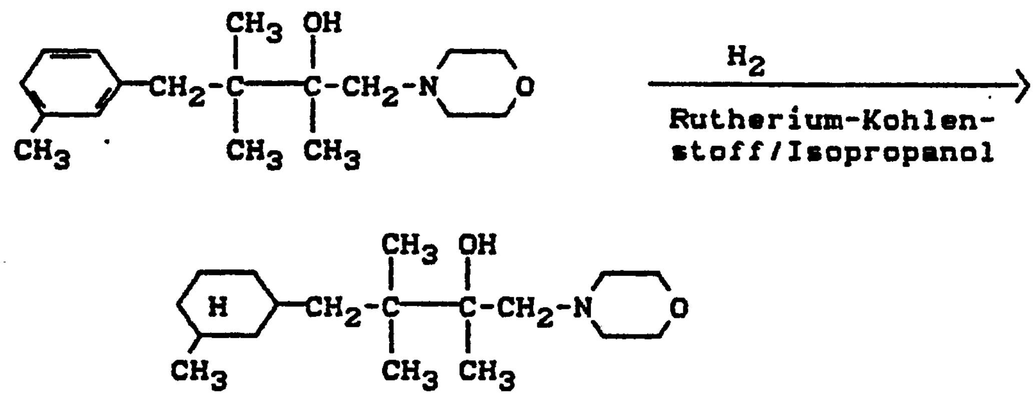


Die zur Durchführung des erfindungsgemäßen Verfahrens (a) als Ausgangsstoffe benötigten Epoxide sind durch die Formel (II) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) für diese Reste genannt wurden.

Die Epoxide der Formel (II) sind bekannt (vgl. z.B. DE-OS 3 413 996) bzw. sie können in allgemein bekannter Art und Weise erhalten werden, indem man Methylketone der Formel (IV)

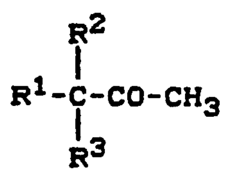
(IV)

in welcher
$R^1$, $R^2$ und $R^3$ die oben angegebene Bedeutung haben,
entweder

α) mit Dimethyloxosulfonium-methylid der Formel

$$\overset{\delta^+\delta^-}{(CH_3)_2SOCH_2} \qquad (V)$$

in an sich bekannter Weise in Gegenwart eines Verdünnungsmittels, wie beispielsweise Dimethylsulfoxid, bei Temperaturen zwischen 20° C und 80° C umsetzt (vgl. hierzu die Angaben in J.Am.Chem.Soc. 87, 1363-1364 (1965)), oder

β) mit Trimethylsulfonium-methylsulfat der Formel
$[(CH_3)_3S^+]\ CH_3SO_4^-$ (VI)
in an sich bekannter Weise in Gegenwart eines inerten organischen Lösungsmittels, wie Acetonitril, und in Gegenwart einer Base, wie z.B. Natriummethylat, bei Temperaturen zwischen 0° C bis 60° C, vorzugsweise bei Raumtemperatur, umsetzt (vgl. auch die Angaben in Heterocycles 8, 397 (1977)).

Die Methylketone der Formel (IV) sind bekannt (vgl. z.B. DE-OS 3 048 266 und DE-OS 3 210 725) bzw. sie können nach den dort angegebenen Verfahren erhalten werden.

Die Epoxide der Formel (II) können auch erhalten werden, indem man entsprechende Olefine nach prinzipiell bekannten Verfahren epoxidiert, wie beispielsweise durch Umsetzung mit Wasserstoffperoxid oder mit Persäuren, gegebenenfalls in Gegenwart eines Lösungsmittels und gegebenenfalls in Gegenwart eines Katalysators bei Temperaturen zwischen –10° C und +300° C.

Die außerdem für das erfindungsgemäße Verfahren (a) als Ausgangsstoffe benötigten Amine sind durch die Formel (III) allgemein definiert. In dieser Formel haben $R^4$ und $R^5$ vorzugsweise diejenigen Bedeutungen, die bereits im Zusammenhang mit der Beschreibung der erfindungsgemäßen Stoffe der Formel (I) für diese Reste genannt wurden.

Die Amine der Formel (III) sind allgemein bekannte Verbindungen der organischen Chemie.

Die zur Durchführung des erfindungsgemäßen Verfahrens (b) als Ausgangsstoffe benötigten Hydroxypropylamin-Derivate sind durch die Formel (Ib) allgemein definiert. In dieser Formel haben $R^2$, $R^3$, $R^4$ und $R^5$ vorzugsweise diejenigen Bedeutungen, die bereits im Zusammenhang mit der Beschreibung

der erfindungsgemäßen Stoffe der Formel (I) für diese Reste genannt wurden. $R^{1-2}$ steht vorzugsweise für jeweils gegebenenfalls einfach oder mehrfach, gleich oder verschieden substituiertes Phenyl, Phenoxy, Phenylalkyl oder Phenoxyalkyl mit jeweils 1 oder 2 Kohlenstoffatomen im Alkylteil, wobei als Phenylsubstituenten jeweils infrage kommen: geradkettiges oder verzweigtes Alkyl, Alkoxy oder Alkylthio mit jeweils bis zu 5 Kohlenstoffatomen; Halogenalkyl, Halogenalkoxy oder Halogenalkylthio mit jeweils 1 oder 2 Kohlenstoffatomen und 1 bis 5 gleichen oder verschiedenen Halogenatomen, wie vorzugsweise Fluor oder Chloratome.

Die Hydroxypropylamin-Derivate der Formel (Ib) sind erfindungsgemäße Stoffe und gemäß Verfahren (a) erhältlich.

Das erfindungsgemäße Verfahren (a) zur Herstellung der neuen substituierten Hydroxypropylamin-Derivate der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol, n- und i-Propanol oder Butanol, Nitrile wie z. B. Acetonitril und Propionitril, Amide wie z. B. Dimethylformamid, sowie Dimethylsulfoxid, Tetramethylensulfon und Hexamethylphosphorsäuretriamid. Verwendet man flüssige Amine der Formel (III) als Ausgangsprodukte, so können diese gleichzeitig als Lösungsmittel fungieren, sofern man sie in einem ausreichenden Überschuß einsetzt.

Das erfindungsgemäße Verfahren (a) kann gegebenenfalls in Gegenwart von Katalysatoren durchgeführt werden. Vorzugsweise infrage kommen Alkalimetallhydroxide wie z.B. Natrium- und Kaliumhydroxid, Alkalicarbonate und -alkoholate wie Natrium- und Kaliumcarbonat, Natrium- und Kaliummethyllat bzw. -ethylat, Kalium-tert.-butylat, ferner Metallsalze der Amine der Formel (III) sowie Carbonsäuren wie z.B. Essigsäure und Propionsäure und Sulfonsäuren wie z.B. Trifluormethansulfonsäure.

Die Reaktionstemperaturen können bei dem erfindungsgemäßen Verfahren (a) in einem größeren Bereich variiert werden. Im allgemeinen arbeitet man bei Temperaturen zwischen –10°C und +250°C, vorzugsweise bei Temperaturen zwischen +40°C und +200°C.

Das erfindungsgemäße Verfahren (a) wird im allgemeinen unter Normaldruck durchgeführt. Es ist jedoch auch möglich, unter erhöhtem Druck zu arbeiten. Unter Druck arbeitet man im allgemeinen zwischen 1,5 und 5 atm., vorzugsweise zwischen 1,5 und 3 atm.

Bei der Durchführung des erfindungsgemäßen Verfahrens (a) setzt man auf 1 Mol Epoxid der Formel (II) vorzugsweise 1 bis 30 Mol Amin der Formel (III), je nachdem ob das Amin auch als Verdünnungsmittel verwendet wird und gegebenenfalls 0,1 bis 10 Mol, vorzugsweise 0,1 Mol bis 1 Mol Katalysator, ein. Die Isolierung der Endprodukte erfolgt nach üblichen Methoden.

Als Verdünnungsmittel kommen bei der Durchführung des erfindungsgemäßen Verfahrens (b) alle unter Hydrierungsbedingungen inerten Solventien in Betracht. Vorzugsweise verwendbar sind Kohlenwasserstoffe wie Petrolether, Pentan, Hexan, Heptan, Cyclohexan, Alkohole wie Methanol, Ethanol, n-Propanol und i-Propanol oder Ether wie Diethylether, Dioxan, Tetrahydrofuran oder Ethylenglykoldimethyl- oder -diethylether. Es kann jedoch auch ohne Verdünnungsmittel gearbeitet werden.

Als Hydrierungsmittel kommen bei der Durchführung des erfindungsgemäßen Verfahrens (b) diejenigen Verbindungen infrage, die Aromaten hydrieren können. Vorzugsweise verwendet man molekularen Wasserstoff.

Als Hydrierungskatalysatoren können bei dem erfindungsgemäßen Verfahren (b) alle üblicherweise für derartige Reaktionen verwendbaren Katalysatoren eingesetzt werden. Vorzugsweise verwendet man Raney-Nickel oder Edelmetallkatalysatoren wie Palladium, Ruthenium, Palladiumoxid, Platin oder Platinoxid, gegebenenfalls auf einem geeigneten Trägerstoff, wie z.B. Kohle.

Die Reaktionstemperaturen können bei dem erfindungsgemäßen Verfahren (b) in einem größeren Bereich variiert werden. Im allgemeinen arbeitet man bei Temperaturen zwischen 50°C und 250°C, vorzugsweise bei Temperaturen zwischen 80°C und 200°C.

Das erfindungsgemäße Verfahren (b) kann bei Normaldruck, aber auch bei erhöhtem Druck durchgeführt werden. Bevorzugt wird unter erhöhtem Druck in Gegenwart von Wasserstoff gearbeitet. Im allgemeinen arbeitet man bei Druckverhältnissen zwischen 5 und 300 atm, vorzugsweise zwischen 10 und 200 atm.

Bei der Durchführung des erfindungsgemäßen Verfahrens (b) setzt man auf 1 Mol Hydroxypropylamin-Derivat der Formel (Ib) im allgemeinen 3,0 bis 30 Mol Hydrierungsmittel und 0,001 bis 0,1 Mol, vorzugsweise 0,01 bis 0,1 Mol Katalysator ein.

Bei der Durchführung des erfindungsgemäßen Verfahrens (b) geht man im allgemeinen so vor, daß man die Verbindungen der Formel (Ib) in Gegenwart eines Verdünnungsmittels, bei der jeweils gewünschten Temperatur mit Wasserstoff im Autoklaven umsetzt. Die Aufarbeitung geschieht nach üblichen Methoden.

Zur Herstellung von Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure,

Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe des Periodensystems der Elemente infrage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschschten Mikroorganismen praktisch eingesetzt werden.

Die Wirkstoffe sind für den Gebrauch als Schädlingsbekämpfungsmittel, vor allem als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophtora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Sepotria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die neuen Wirkstoffe können mit besonders gutem Erfolg zur Bekämpfung von Getreidekrankheiten, wie beispielsweise gegen den Erreger des Gerstenmehltaus (Erysiphe graminis) eingesetzt werden. Darüberhinaus zeigen die Verbindungen der Formel (I) eine sehr breite fungizide Wirkung z.B. gegen den Erreger der Fleckenkrankheit am Reis (Pyricularia oryzae).

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Geweichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02% am Wirkungsort erforderlich.

Herstellungsbeispiele

Beispiel 1

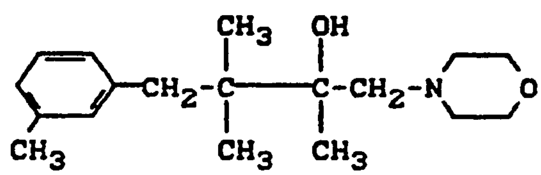

(Verfahren a)

Zu 6,1 g (0,07 Mol) Morpholin in 40 ml n-Butanol und 0,2 ml Eisessig werden 14 g (0,069 Mol) rohes 3,3-Dimethyl-2-methyl-4-(3-methyl-phenyl)-buten-1-oxid in 20 ml n-Butanol zugetropft und 17 Stunden unter Rückfluß erhitzt. Anschließend wird das Lösungsmittel im Wasserstrahlvakuum abdestilliert. Der Rückstand wird über eine Kieselgelsäule nacheinander mit den Elutionsmitteln Methylenchlorid und Methylenchlorid/Methanol (9:1) chromatographiert.

Man erhält 11,4 g (57 % der Theorie) 3,3-Dimethyl-2-methyl-4-(3-methyl-phenyl)-1-(morpholin-4-yl)-2-butanol als Öl vom Brechungsindex $n_D^{20}$: 1,5215.

Die Struktur wird durch NMR-Spektren und die Reinheit durch Gaschromatographie gesichert.

Beispiel 2

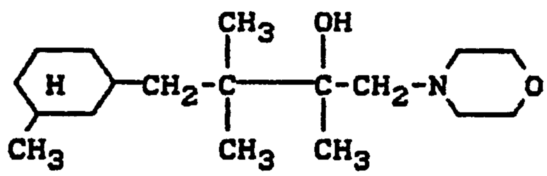

(Verfahren b)

6 g (0,02 Mol) 3,3-Dimethyl-2-methyl-4-(3-methyl-phenyl)-1-(morpholin-4-yl)-2-butanol (Beispiel 1) werden in 100 ml Isopropanol gelöst und mit 2 g 5%igem Ruthenium-Kohlenstoff bei 130°C und 200 atm innerhalb von 1 Stunde hydriert. Der Katalysator wird abfiltriert und das Lösungsmittel im Wasserstrahlvakuum abdestilliert.

Man erhält 4,8 g (77% der Theorie) 3,3-Dimethyl-2-methyl-4-(3-methyl-cyclohexyl)-1-(morpholin-4-yl)-2-butanol vom Brechungsindex $n_D^{20}$ : 1,4852.

Die Struktur wird durch NMR-Spektren und die Reinheit durch Gaschromatographie gesichert.

Analog den Beispielen 1 und 2 bzw. gemäß den Verfahrensvarianten (a) und (b) können die folgenden Verbindungen der Formel (I)

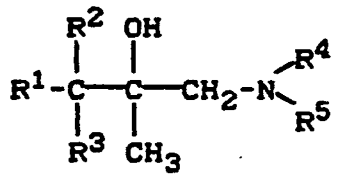 (I)

erhalten werden:

Tabelle 2

| Bsp.Nr. | $R^1$ | $R^2$ | $R^3$ | 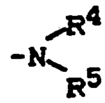 | Physik. Konst. |
|---|---|---|---|---|---|
| 3 | 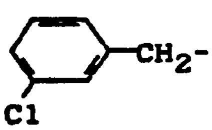 | $CH_3$ | $CH_3$ | 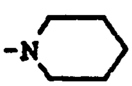 | $n_D^{20}$ :1,5302 |
| 4 | 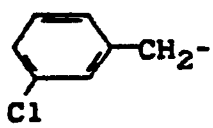 | $CH_3$ | $CH_3$ | 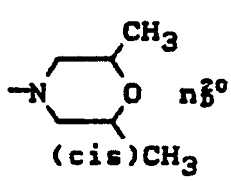 | $n_D^{20}$ :1,5221 |

<u>Tabelle 2</u> (Fortsetzung)

| Bsp.Nr. | $R^1$ | $R^2$ | $R^3$ | 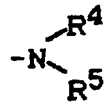 | Physik. Konst. |
|---|---|---|---|---|---|
| 5 | 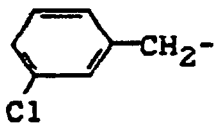 | $CH_3$ | $CH_3$ | 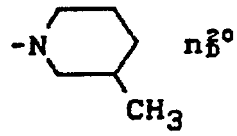 | $n_D^{20}$ :1,5261 |
| 6 | 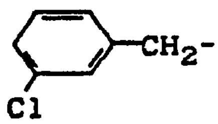 | $CH_3$ | $CH_3$ | 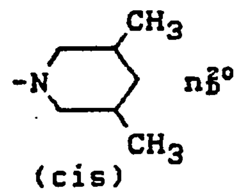 (cis) | $n_D^{20}$ :1,5205 |
| 7 | 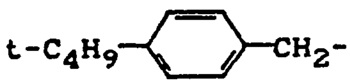 | $CH_3$ | $CH_3$ | 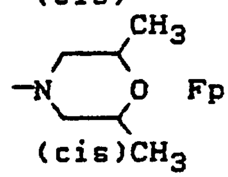 | Fp: 68° C |
| 8 | 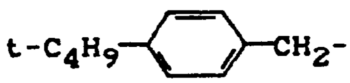 | $CH_3$ | $CH_3$ | 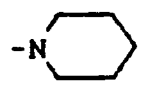 | Fp:55-57° C |
| 9 | 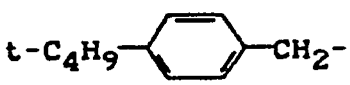 | $CH_3$ | $CH_3$ | 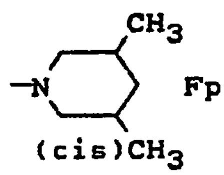 | Fp:65° C |
| 10 | 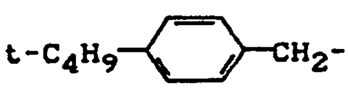 | $CH_3$ | $CH_3$ | 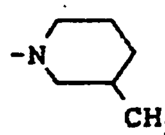 | $n_D^{20}$ :1,5079 |
| 11 | 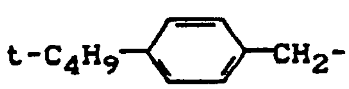 | $CH_3$ | $CH_3$ | 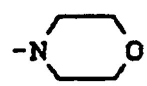 | Fp:89° C |
| 12 | 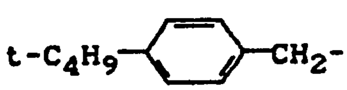 | H | $CH_3$ | 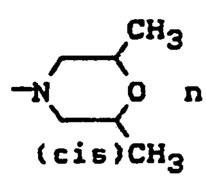 | $n_D^{20}$ :1,5082 |

Tabelle 2 (Fortsetzung)

| Bsp.Nr. | $R^1$ | $R^2$ | $R^3$ | 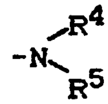 | Physik. Konst. |
|---|---|---|---|---|---|
| 13 | 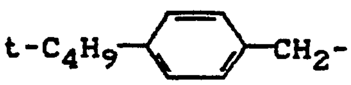 | H | $CH_3$ | 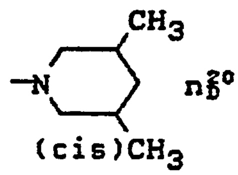 (cis) | $n_D^{20}$ :1,5059 |
| 14 | 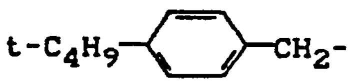 | H | $CH_3$ | 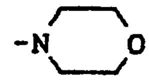 | $n_D^{20}$ :1,5176 |
| 15 | 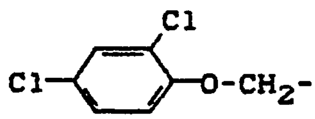 | $CH_3$ | $CH_3$ | (cis) 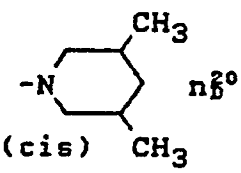 | $n_D^{20}$ :1,5240 |
| 16 | 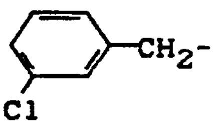 | H | $C_2H_5$ | 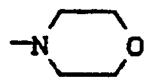 | $n_D^{20}$ :1,5263 |
| 17 | 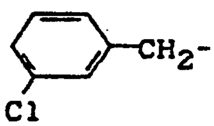 | H | $C_2H_5$ | (cis) 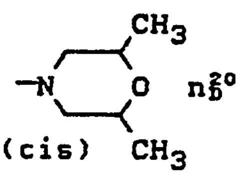 | $n_D^{20}$ :1,5111 |
| 18 | 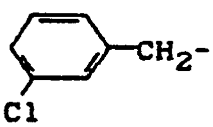 | H | $C_2H_5$ | 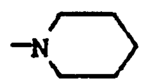 | $n_D^{20}$ :1,5231 |
| 19 | 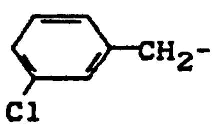 | H | $C_2H_5$ | 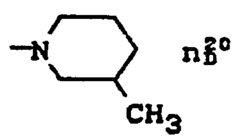 | $n_D^{20}$ :1,5175 |
| 20 | 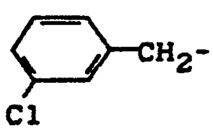 | H | $C_2H_5$ | 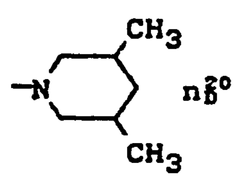 | $n_D^{20}$ :1,5124 |

<u>Tabelle 2</u> (Fortsetzung)

| Bsp.Nr. | $R^1$ | $R^2$ | $R^3$ | $-N(R^4)R^5$ | Physik. Konst. |
|---|---|---|---|---|---|
| 21 | 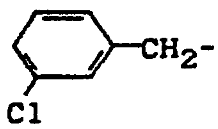 | H | $C_2H_5$ | 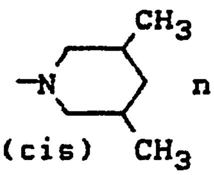 (cis) | $n_D^{20}$ :1,5135 |
| 22 | 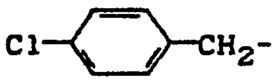 | H | $C_2H_5$ | 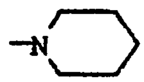 | $n_D^{20}$ :1,5213 |
| 23 | 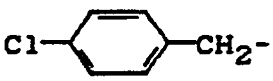 | H | $C_2H_5$ | 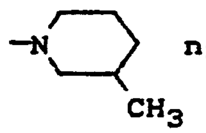 | $n_D^{20}$ :1,5167 |
| 24 | 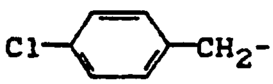 | H | $C_2H_5$ | 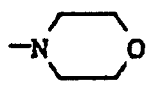 | $n_D^{20}$ :1,5264 |
| 25 | 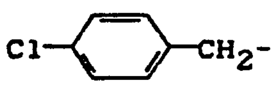 | H | $C_2H_5$ | 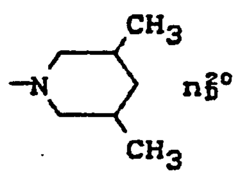 | $n_D^{20}$ :1,5126 |
| 26 | 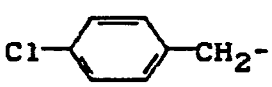 | H | $C_2H_5$ | 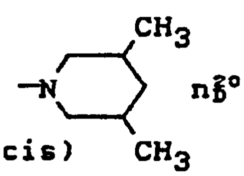 (cis) | $n_D^{20}$ :1,5134 |
| 27 | 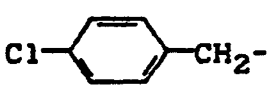 | H | $C_2H_5$ | 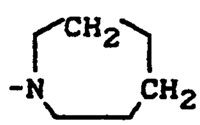 | $n_D^{20}$ :1,5250 |

Tabelle 2 (Fortsetzung)

| Bsp.Nr. | $R^1$ | $R^2$ | $R^3$ | 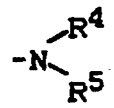 | Physik. Konst. |
|---|---|---|---|---|---|
| 28 | 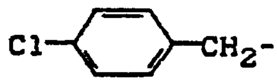 | H | $C_2H_5$ | 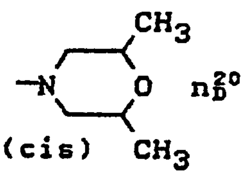 (cis) | $n_D^{20}$ :1,5137 |
| 29 | 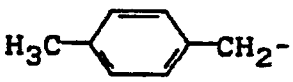 | H | $CH_3$ | 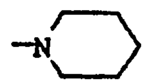 | $n_D^{20}$ :1,5123 |
| 30 | 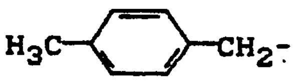 | H | $CH_3$ | 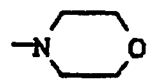 | $n_D^{20}$ :1,5171 |
| 31 | 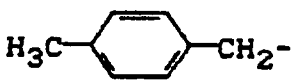 | H | $CH_3$ | 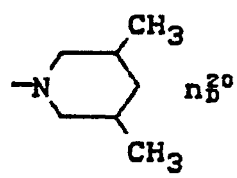 | $n_D^{20}$ :1,5049 |
| 32 | 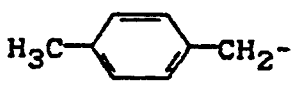 | H | $CH_3$ | 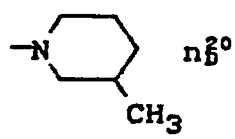 | $n_D^{20}$ :1,5088 |
| 33 | 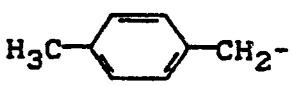 | H | $CH_3$ | 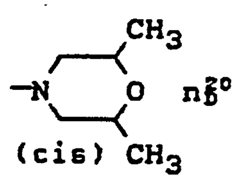 (cis) | $n_D^{20}$ :1,5055 |
| 34 | 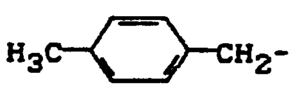 | H | $CH_3$ | 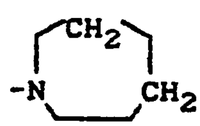 | $n_D^{20}$ :1,5162 |
| 35 | 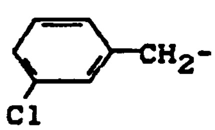 | H | $CH_3$ | 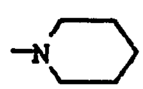 | $n_D^{20}$ :1,5256 |

Tabelle 2 (Fortsetzung)

| Bsp.Nr. | $R^1$ | $R^2$ | $R^3$ | $-N\begin{matrix}R^4\\R^5\end{matrix}$ | Physik. Konst. |
|---|---|---|---|---|---|
| 36 | 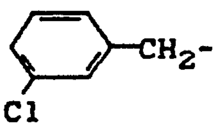 | H | $CH_3$ | 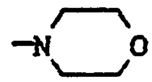 | $n_D^{20}$ :1,5300 |
| 37 | 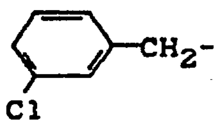 | H | $CH_3$ | 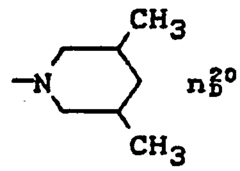 | $n_D^{20}$ :1,5148 |
| 38 | 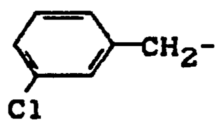 | H | $CH_3$ | 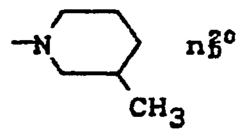 | $n_D^{20}$ :1,5187 |
| 39 | 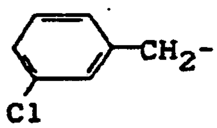 | H | $CH_3$ | 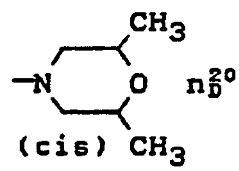 (cis) | $n_D^{20}$ :1,5166 |
| 40 | 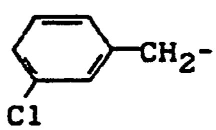 | H | $CH_3$ | 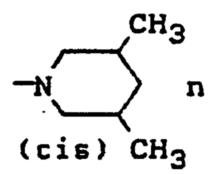 (cis) | $n_D^{20}$ :1,5149 |
| 41 | 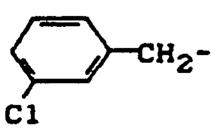 | H | $-CH_3$ | 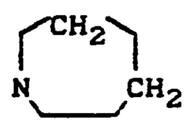 | $n_D^{20}$ :1,5272 |
| 42 | 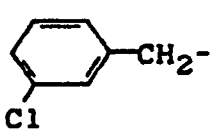 | H | $CH_3$ | 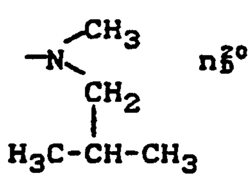 | $n_D^{20}$ :1,5044 |

Tabelle 2 (Fortsetzung)

| Bsp.Nr. | $R^1$ | $R^2$ | $R^3$ | 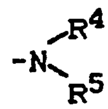 | Physik. Konst. |
|---|---|---|---|---|---|
| 43 | 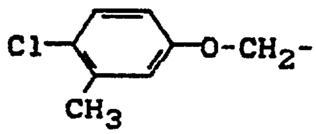 | $CH_3$ | $CH_3$ | 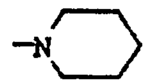 | $n_D^{20}$ :1,5256 |
| 44 | 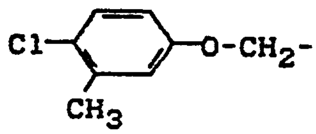 | $CH_3$ | $CH_3$ | 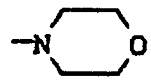 | $n_D^{20}$ :1,5239 |
| 45 | 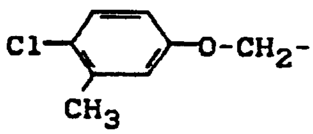 | $CH_3$ | $CH_3$ | 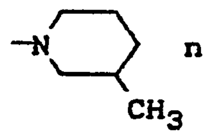 | $n_D^{20}$ :1,5213 |
| 46 | 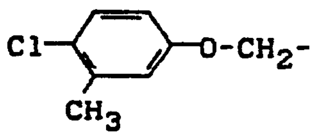 | $CH_3$ | $CH_3$ | 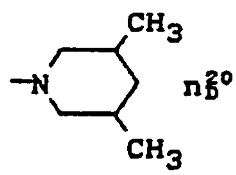 | $n_D^{20}$ :1,5169 |
| 47 | 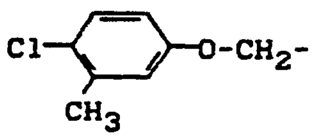 | $CH_3$ | $CH_3$ | 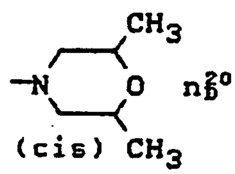 (cis) | $n_D^{20}$ :1,5138 |
| 48 | 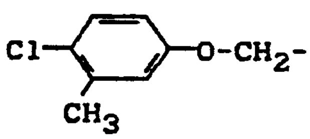 | $CH_3$ | $CH_3$ | 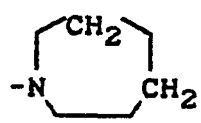 | $n_D^{20}$ :1,5278 |
| 49 | 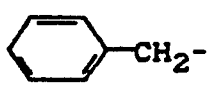 | $CH_3$ | $CH_3$ | 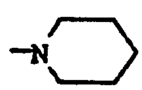 | Fp:54-56° C |
| 50 | 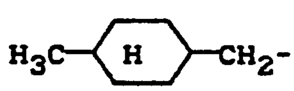 | H | $CH_3$ | 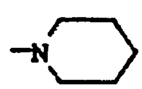 | $n_D^{20}$ :1,4806 |

Tabelle 2 (Fortsetzung)

| Bsp.Nr. | $R^1$ | $R^2$ | $R^3$ | 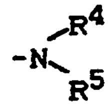 | Physik. Konst. |
|---|---|---|---|---|---|
| 51 | 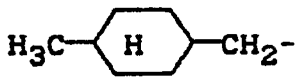 | H | $CH_3$ | 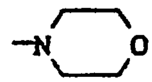 | $n_D^{20}$ :1,4825 |
| 52 | 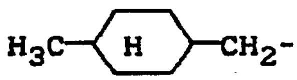 | H | $CH_3$ | 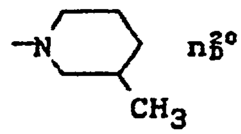 | $n_D^{20}$ :1,4774 |
| 53 | 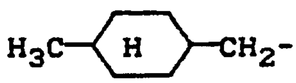 | H | $CH_3$ | 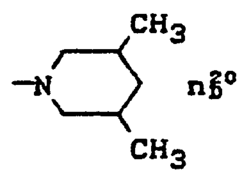 | $n_D^{20}$ :1,4760 |
| 54 | 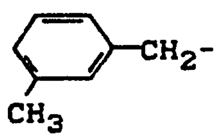 | $CH_3$ | $CH_3$ | 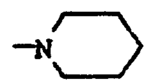 | $n_D^{20}$ :1,5185 |
| 55 | 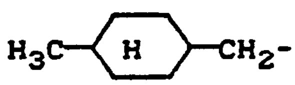 | H | $CH_3$ | 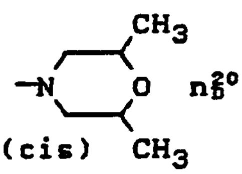 (cis) | $n_D^{20}$ :1,4746 |
| 56 | 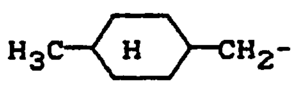 | H | $-CH_3$ | 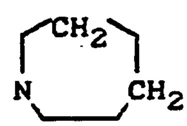 | $n_D^{20}$ :1,4841 |
| 57 | 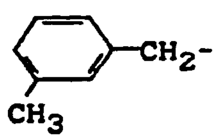 | $CH_3$ | $CH_3$ | 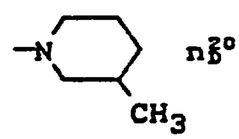 | $n_D^{20}$ :1,5134 |
| 58 | 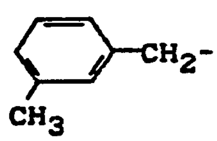 | $CH_3$ | $CH_3$ | 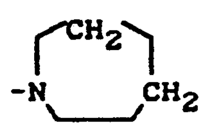 | $n_D^{20}$ :1,5219 |

Tabelle 2 (Fortsetzung)

| Bsp.Nr. | $R^1$ | $R^2$ | $R^3$ | 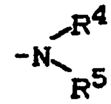 | Physik. Konst. |
|---|---|---|---|---|---|
| 59 | 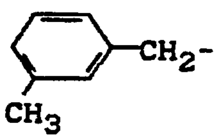 | $CH_3$ | $CH_3$ | 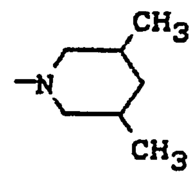 | $n_D^{20}$ :1,5083 |
| 60 | 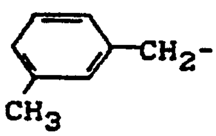 | $CH_3$ | $CH_3$ | 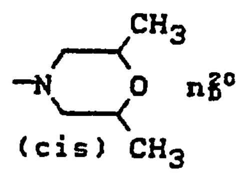 (cis) | $n_D^{20}$ :1,5097 |
| 61 | 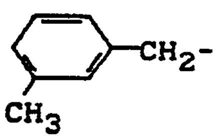 | $CH_3$ | $CH_3$ | 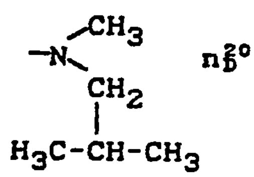 | $n_D^{20}$ :1,5001 |
| 62 | 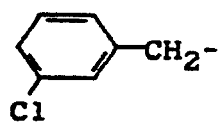 | H | $C_2H_5$ | 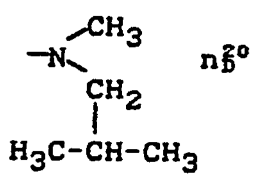 | $n_D^{20}$ :1,5032 |
| 63 | 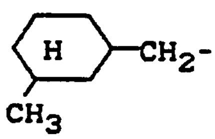 | $CH_3$ | $CH_3$ | 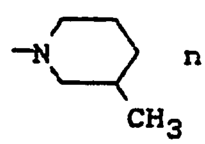 | $n_D^{20}$ :1,4886 |
| 64 | 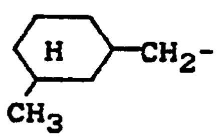 | $CH_3$ | $CH_3$ | 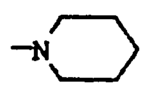 | $n_D^{20}$ :1,4844 |
| 65 | 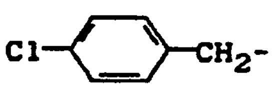 | H | $C_2H_5$ | 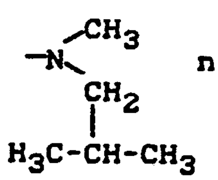 | $n_D^{20}$ :1,5034 |

Tabelle 2 (Fortsetzung)

| XL1,0 Bsp.Nr. | $R^1$ | $R^2$ | $R^3$ | 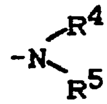 | Physik. Konst. |
|---|---|---|---|---|---|
| 66 | 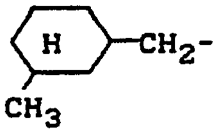 | $CH_3$ | $CH_3$ | 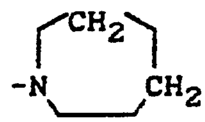 | $n_D^{20}$ :1,4818 |
| 67 | 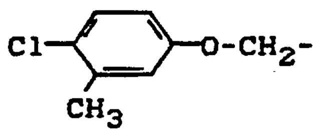 | $CH_3$ | $CH_3$ | 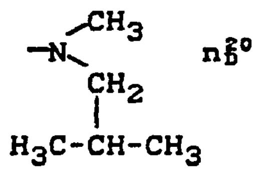 | $n_D^{20}$ :1,5067 |
| 68 | 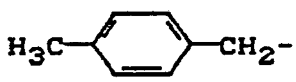 | H | $CH_3$ | 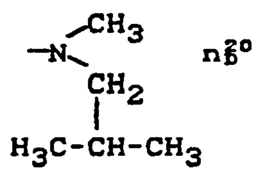 | $n_D^{20}$ :1,4967 |
| 69 | 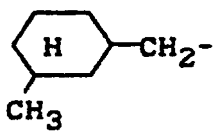 | $CH_3$ | $CH_3$ | 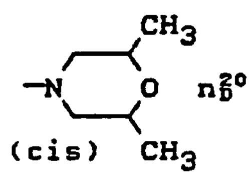 | $n_D^{20}$ :1,4777 |
| 70 | 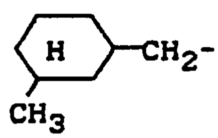 | $CH_3$ | $CH_3$ | 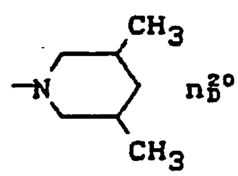 | $n_D^{20}$ :1,4788 |
| 71 | 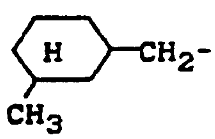 | $CH_3$ | $CH_3$ | 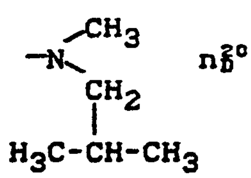 | $n_D^{20}$ :1,4678 |
| 72 | 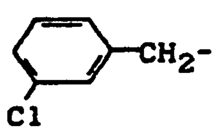 | $CH_3$ | $CH_3$ | 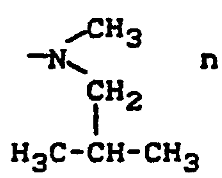 | $n_D^{20}$ :1,5069 |

Tabelle 2 (Fortsetzung)

| Bsp.Nr. | $R^1$ | $R^2$ | $R^3$ | $-N(R^4)(R^5)$ | Physik. Konst. |
|---|---|---|---|---|---|
| 73 | 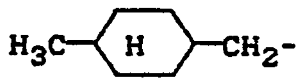 | H | $CH_3$ | 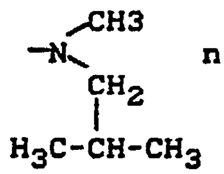 | $n_D^{20}$ :1,4646 |
| 74 | 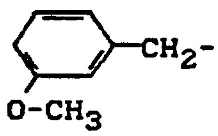 | H | $CH_3$ | 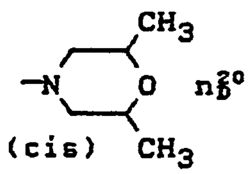 (cis) | $n_D^{20}$ :1,5143 |
| 75 | 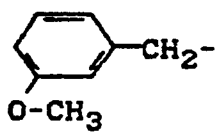 | H | $CH_3$ | 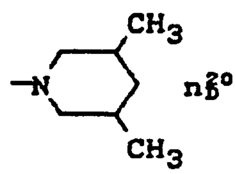 | $n_D^{20}$ :1,5120 |
| 76 | 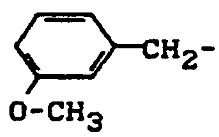 | H | $CH_3$ | 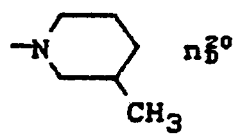 | $n_D^{20}$ :1,5159 |
| 77 | 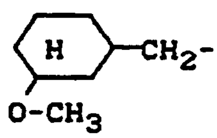 | H | $CH_3$ | 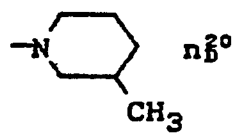 | $n_D^{20}$ :1,4756 |
| 78 | 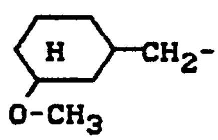 | H | $CH_3$ | 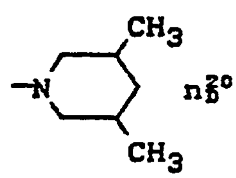 | $n_D^{20}$ :1,4759 |
| 79 | 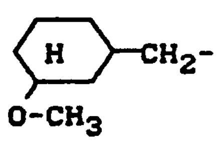 | H | $CH_3$ | 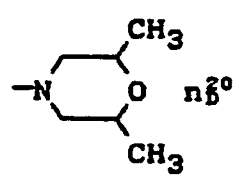 | $n_D^{20}$ :1,4776 |

<u>Tabelle 2</u> (Fortsetzung)

| Bsp.Nr. | $R^1$ | $R^2$ | $R^3$ | 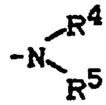 | Physik. Konst. |
|---|---|---|---|---|---|
| 80 | 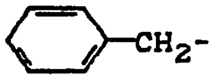 | $CH_3$ | $CH_3$ | 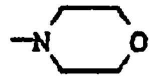 | $n_D^{20}$ :1,5222 |
| 81 | 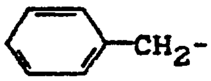 | $CH_3$ | $CH_3$ | 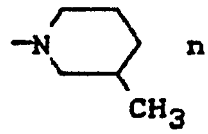 | $n_D^{20}$ :1,5122 |
| 82 | 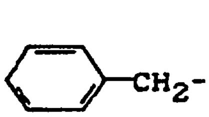 | $CH_3$ | $CH_3$ | 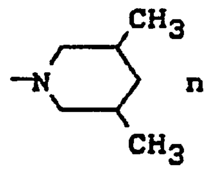 | $n_D^{20}$ :1,5093 |
| 83 | 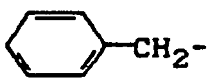 | $CH_3$ | $CH_3$ | 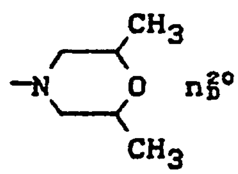 | $n_D^{20}$ :1,5103 |
| 84 | 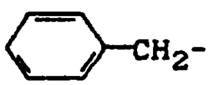 | $CH_3$ | $CH_3$ | 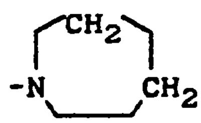 | $n_D^{20}$ :1,5216 |
| 85 | 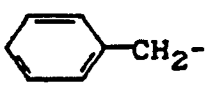 | $CH_3$ | $CH_3$ | 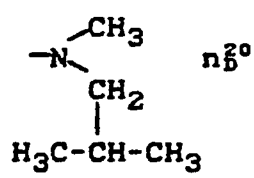 | $n_D^{20}$ :1,4990 |
| 86 | 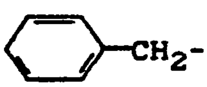 | $CH_3$ | $CH_3$ | 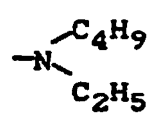 | $n_D^{20}$ :1,4998 |
| 87 | 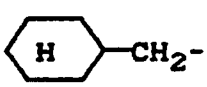 | $CH_3$ | $CH_3$ | 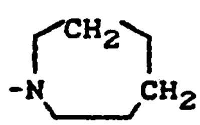 | $n_D^{20}$ :1,4912 |

Tabelle 2 (Fortsetzung)

| Bsp.Nr. | $R^1$ | $R^2$ | $R^3$ | 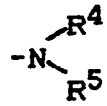 | Physik. Konst. |
|---|---|---|---|---|---|
| 88 | 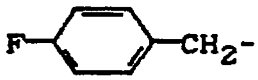 | H | $CH_3$ | 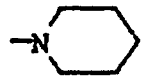 | $n_D^{20}$ :1,5037 |
| 89 | 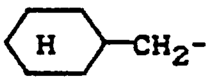 | $CH_3$ | $CH_3$ | 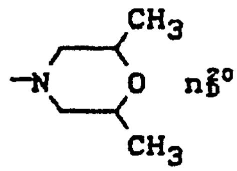 | $n_D^{20}$ :1,4796 |
| 90 | 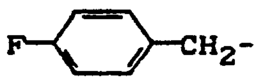 | H | $CH_3$ | 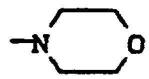 | $n_D^{20}$ :1,5082 |
| 91 | 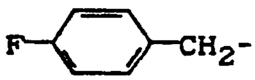 | H | $CH_3$ | 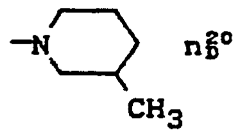 | $n_D^{20}$ :1,4985 |
| 92 | 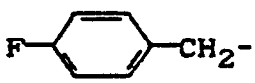 | H | $CH_3$ | 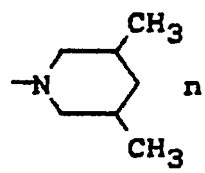 | $n_D^{20}$ :1,4958 |
| 93 | 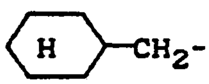 | $CH_3$ | $CH_3$ | 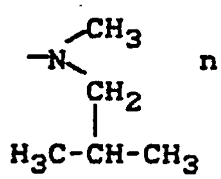 | $n_D^{20}$ :1,4694 |
| 94 | 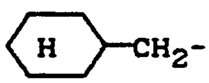 | $CH_3$ | $CH_3$ | 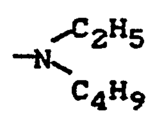 | $n_D^{20}$ :1,4714 |
| 95 | 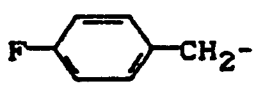 | H | $CH_3$ | 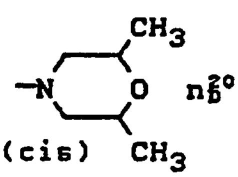 (cis) | $n_D^{20}$ :1,4976 |

Tabelle 2 (Fortsetzung)

| Bsp.Nr. | $R^1$ | $R^2$ | $R^3$ | 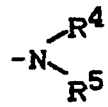 | Physik. Konst. |
|---|---|---|---|---|---|
| 96 | 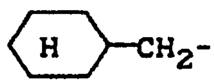 | $CH_3$ | $CH_3$ | 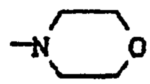 | $n_D^{20}$ :1,4891 |
| 97 | 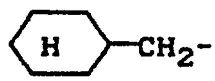 | $CH_3$ | $CH_3$ | 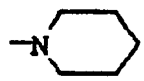 | $n_D^{20}$ :1,4870 |
| 98 | 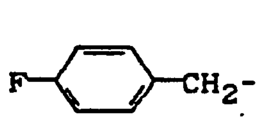 | H | $CH_3$ | 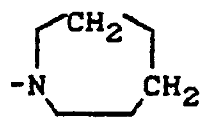 | $n_D^{20}$ :1,5071 |
| 99 | 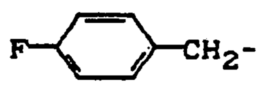 | H | $CH_3$ | 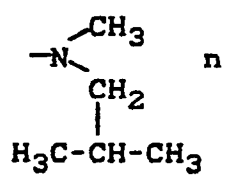 | $n_D^{20}$ :1,4839 |
| 100 | 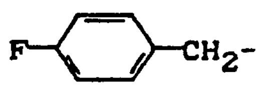 | H | $CH_3$ | 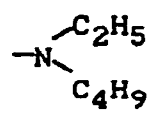 | $n_D^{20}$ :1,4847 |
| 101 | 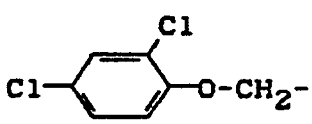 | $CH_3$ | $CH_3$ | 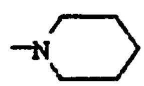 | $n_D^{20}$ :1,5329 |
| 102 | 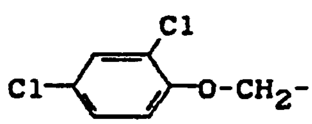 | $CH_3$ | $CH_3$ | 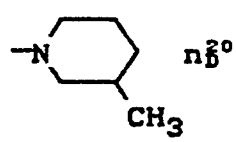 | $n_D^{20}$ :1,5281 |
| 103 | 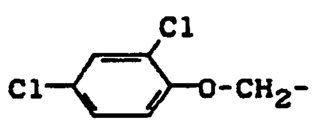 | $CH_3$ | $CH_3$ | 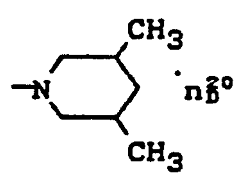 | $n_D^{20}$ :1,5230 |

Tabelle 2 (Fortsetzung)

| Bsp.Nr. | $R^1$ | $R^2$ | $R^3$ | $-N(R^4)(R^5)$ | Physik. Konst. |
|---|---|---|---|---|---|
| 104 | 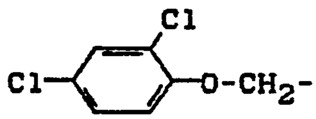 | $CH_3$ | $CH_3$ | 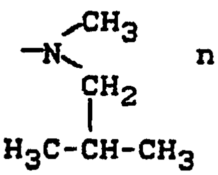 | $n_D^{20}$ :1,5138 |
| 105 | 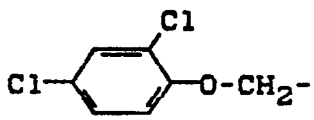 | $CH_3$ | $CH_3$ | 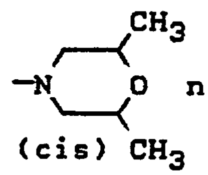 (cis) | $n_D^{20}$ :1,5235 |
| 106 | 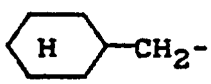 | $CH_3$ | $CH_3$ | 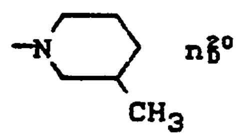 | $n_D^{20}$ :1,4835 |
| 107 | 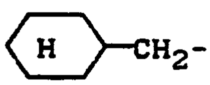 | $CH_3$ | $CH_3$ | 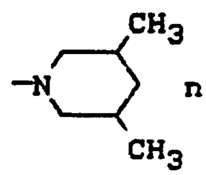 | $n_D^{20}$ :1,4805 |
| 108 | 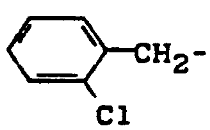 | $CH_3$ | $CH_3$ | 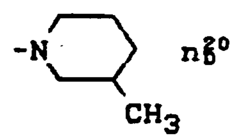 | $n_D^{20}$ :1,5213 |
| 109 | 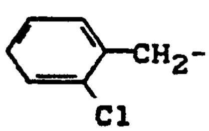 | $CH_3$ | $CH_3$ | 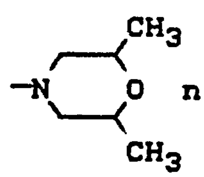 | $n_D^{20}$ :1,5197 |
| 110 | 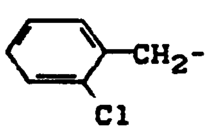 | $CH_3$ | $CH_3$ | 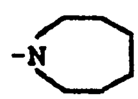 | $n_D^{20}$ :1,5297 |

Tabelle 2 (Fortsetzung)

| Bsp.Nr. | $R^1$ | $R^2$ | $R^3$ | 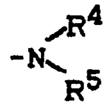 | Physik. Konst. |
|---|---|---|---|---|---|
| 111 | 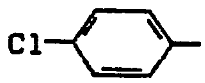 | $CH_3$ | $CH_3$ | 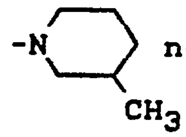 | $n_D^{20}$ :1,5218 |
| 112 | 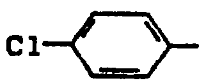 | $CH_3$ | $CH_3$ | 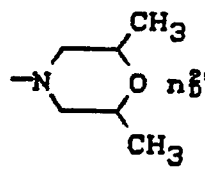 | $n_D^{20}$ :1,5171 |
| 113 | 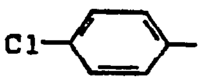 | $CH_3$ | $CH_3$ | 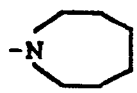 | $n_D^{20}$ :1,5331 |
| 114 | 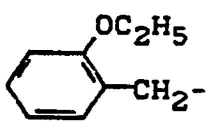 | H | $CH_3$ | 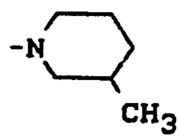 | $n_D^{20}$ :1,5096 |
| 115 | 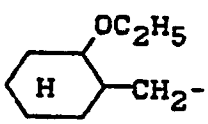 | H | $CH_3$ | 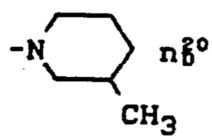 | $n_D^{20}$ :1,4760 |
| 116 | 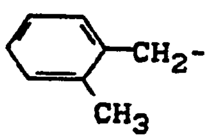 | $CH_3$ | $CH_3$ | 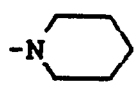 | $n_D^{20}$ :1,5215 |
| 117 | 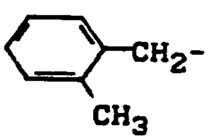 | $CH_3$ | $CH_3$ | 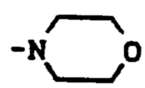 | $n_D^{20}$ :1,5253 |
| 118 | 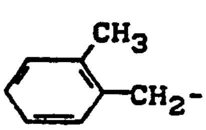 | $CH_3$ | $CH_3$ | 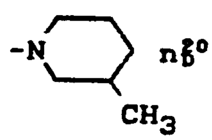 | $n_D^{20}$ :1,5166 |

Tabelle 2 (Fortsetzung)

| Bsp.Nr. | $R^1$ | $R^2$ | $R^3$ | $-N{<}^{R^4}_{R^5}$ | Physik. Konst. |
|---|---|---|---|---|---|
| 119 | 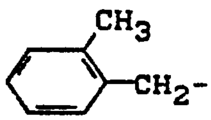 | $CH_3$ | $CH_3$ | 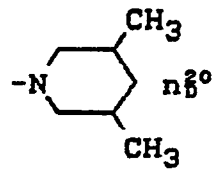 | $n_D^{20}$ :1,5121 |
| 120 | 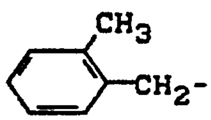 | $CH_3$ | $CH_3$ | 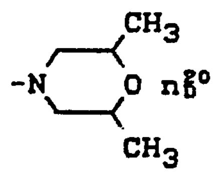 | $n_D^{20}$ :1,5134 |
| 121 | 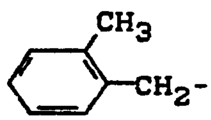 | $CH_3$ | $CH_3$ | 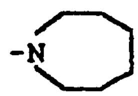 | $n_D^{20}$ :1,5247 |
| 122 | 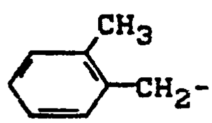 | $CH_3$ | $CH_3$ | $-N(CH_3)-CH_2-CH(CH_3)_2$ | $n_D^{20}$ :1,5035 |
| 123 | 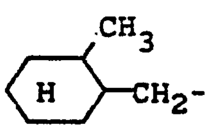 | $CH_3$ | $CH_3$ | 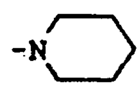 | $n_D^{20}$ :1,4872 |
| 124 | 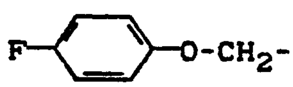 | $CH_3$ | $CH_3$ | 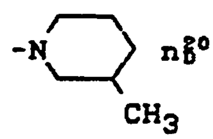 | $n_D^{20}$ :1,4996 |
| 125 | 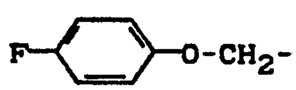 | $CH_3$ | $CH_3$ | 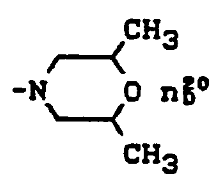 | $n_D^{20}$ :1,4972 |
| 126 | 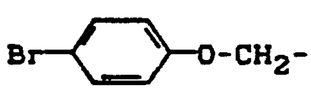 | $CH_3$ | $CH_3$ | 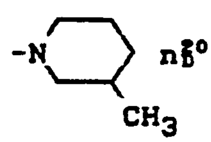 | $n_D^{20}$ :1,5338 |

Tabelle 2 (Fortsetzung)

| Bsp.Nr. | $R^1$ | $R^2$ | $R^3$ | 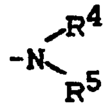 | Physik. Konst. |
|---|---|---|---|---|---|
| 127 | 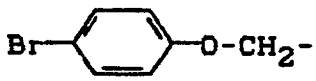 | $CH_3$ | $CH_3$ | 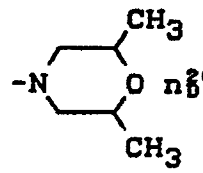 | $n_D^{20}$ :1,5262 |
| 128 | 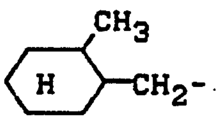 | $CH_3$ | $CH_3$ | 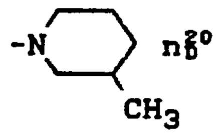 | $n_D^{20}$ :1,4842 |
| 129 | 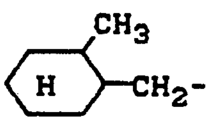 | $CH_3$ | $CH_3$ | 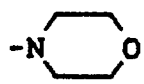 | $n_D^{20}$ :1,4886 |
| 130 | 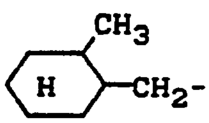 | $CH_3$ | $CH_3$ | 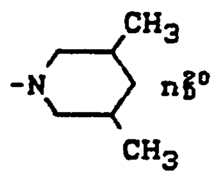 | $n_D^{20}$ :1,4817 |
| 131 | 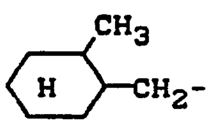 | $CH_3$ | $CH_3$ | 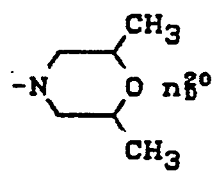 | $n_D^{20}$ :1,4819 |
| 132 | 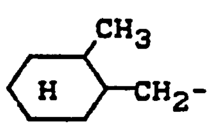 | $CH_3$ | $CH_3$ | 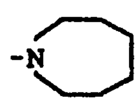 | $n_D^{20}$ :1,4917 |
| 133 | 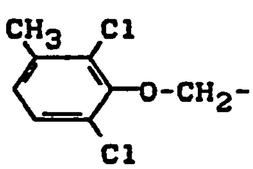 | $CH_3$ | $CH_3$ | 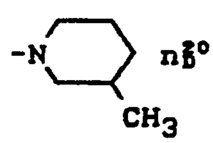 | $n_D^{20}$ :1,5205 |

Tabelle 2 (Fortsetzung)

| Bsp.Nr. | $R^1$ | $R^2$ | $R^3$ | 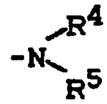 | Physik. Konst. |
|---|---|---|---|---|---|
| 134 | 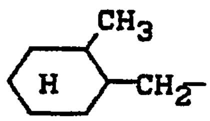 | $CH_3$ | $CH_3$ | 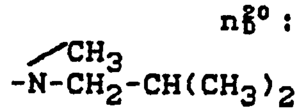 | $n_D^{20}$ :1,4701 |
| 135 | 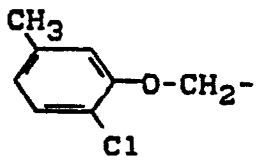 | $CH_3$ | $CH_3$ | 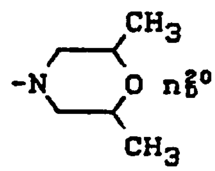 | $n_D^{20}$ :1,5153 |
| 136 | 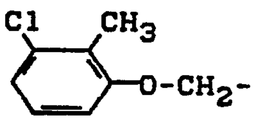 | $CH_3$ | $CH_3$ | 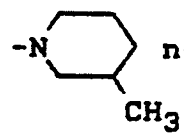 | $n_D^{20}$ :1,5239 |
| 137 | 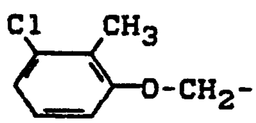 | $CH_3$ | $CH_3$ | 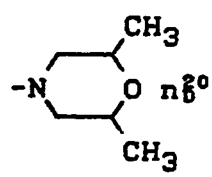 | $n_D^{20}$ :1,5178 |
| 138 | 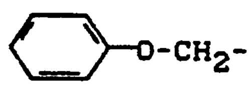 | $CH_3$ | $CH_3$ | 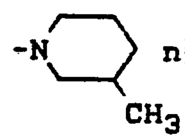 | $n_D^{20}$ :1,5093 |
| 139 | 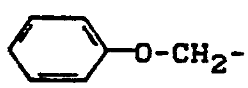 | $CH_3$ | $CH_3$ | 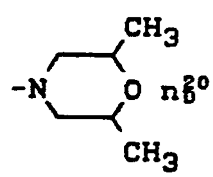 | $n_D^{20}$ :1,5056 |
| 140 | 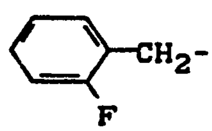 | $CH_3$ | $CH_3$ | 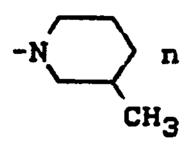 | $n_D^{20}$ :1,5034 |

<u>Tabelle 2</u> (Fortsetzung)

| Bsp.Nr. | $R^1$ | $R^2$ | $R^3$ | $-N(R^4)R^5$ | Physik. Konst. |
|---|---|---|---|---|---|
| 141 | 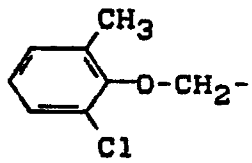 | $CH_3$ | $CH_3$ | 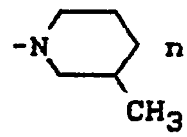 | $n_D^{20}$ : 1,5164 |
| 142 | 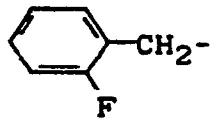 | $CH_3$ | $CH_3$ | 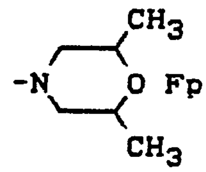 | Fp: 65-68° C |
| 143 | 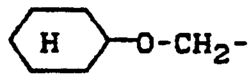 | $CH_3$ | $CH_3$ | 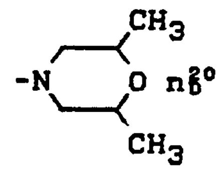 | $n_D^{20}$ : 1,4742 |
| 144 | 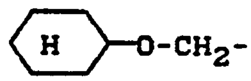 | $CH_3$ | $CH_3$ | 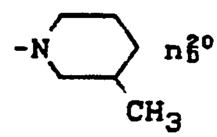 | $n_D^{20}$ : 1,4756 |
| 145 | 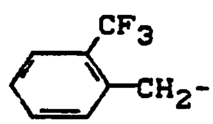 | $CH_3$ | $CH_3$ | 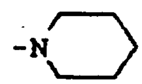 | $n_D^{20}$ : 1,4918 |
| 146 | 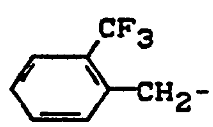 | $CH_3$ | $CH_3$ | 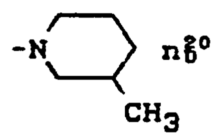 | $n_D^{20}$ : 1,4847 |
| 147 | 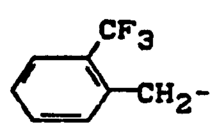 | $CH_3$ | $CH_3$ | 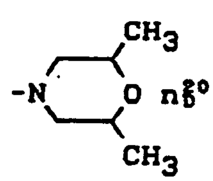 | $n_D^{20}$ : 1,4861 |

Tabelle 2 (Fortsetzung)

| Bsp.Nr. | $R^1$ | $R^2$ | $R^3$ | 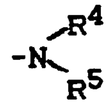 | Physik. Konst. |
|---|---|---|---|---|---|
| 148 | 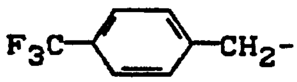 | $CH_3$ | $CH_3$ | 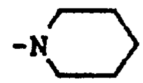 | $n_D^{20}$ : 1,4853 |
| 149 | 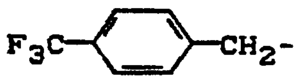 | $CH_3$ | $CH_3$ | 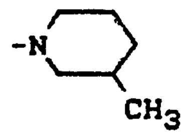 | $n_D^{20}$ : 1,4827 |
| 150 | 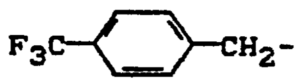 | $CH_3$ | $CH_3$ | 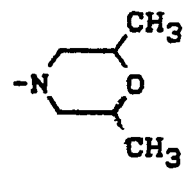 | $n_D^{20}$ : 1,4809 |
| 151 | 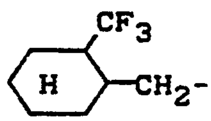 | $CH_3$ | $CH_3$ | 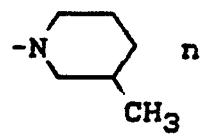 | $n_D^{20}$ : 1,4631 |
| 152 | 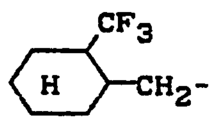 | $CH_3$ | $CH_3$ | 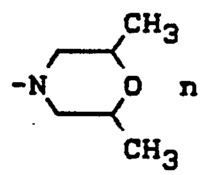 | $n_D^{20}$ : 1,4611 |
| 153 | 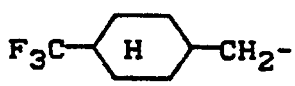 | $CH_3$ | $CH_3$ | 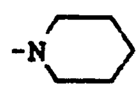 | $n_D^{20}$ : 1,4605 |
| 154 | 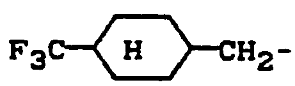 | $CH_3$ | $CH_3$ | 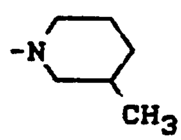 | zähes Öl |
| 155 | 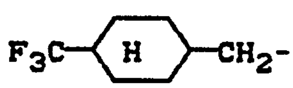 | $CH_3$ | $CH_3$ | 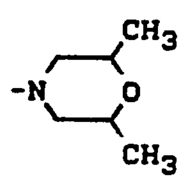 | $n_D^{20}$ : 1,4589 |

Tabelle 2 (Fortsetzung)

| Bsp.Nr. | $R^1$ | $R^2$ | $R^3$ | $-N(R^4)R^5$ | Physik. Konst. |
|---|---|---|---|---|---|
| 156 | 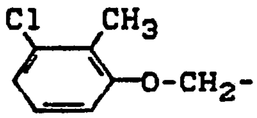 | $CH_3$ | $CH_3$ | 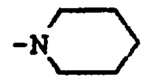 | $n_D^{20}$ :1,5277 |
| 157 | 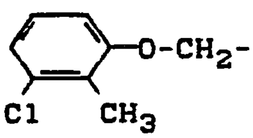 | $CH_3$ | $CH_3$ | 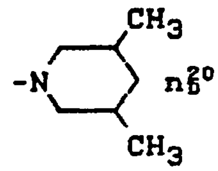 | $n_D^{20}$ :1,5182 |
| 158 | 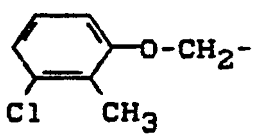 | $CH_3$ | $CH_3$ | 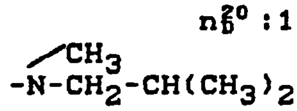 | $n_D^{20}$ :1,5091 |
| 159 | 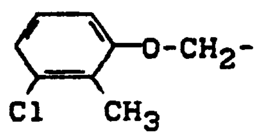 | $CH_3$ | $CH_3$ | 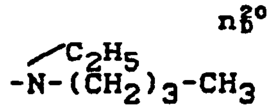 | $n_D^{20}$ :1,5116 |
| 160 | 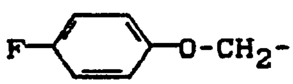 | $CH_3$ | $CH_3$ | 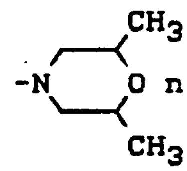 | $n_D^{20}$ :1,4999 |
| 161 | 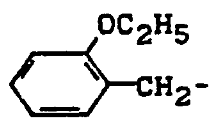 | H | $CH_3$ | 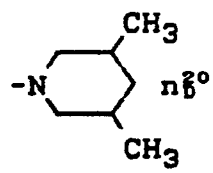 | $n_D^{20}$ :1,5043 |
| 162 | 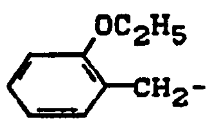 | H | $CH_3$ | 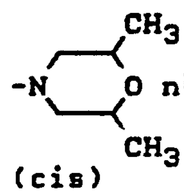 (cis) | $n_D^{20}$ :1,5064 |

Tabelle 2 (Fortsetzung)

| Bsp.Nr. | $R^1$ | $R^2$ | $R^3$ | 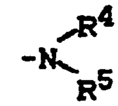 | Physik. Konst. |
|---|---|---|---|---|---|
| 163 | 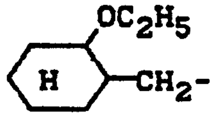 | H | $CH_3$ | 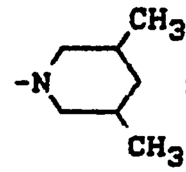 | $n_D^{20}$ : 1,4753 |
| 164 | 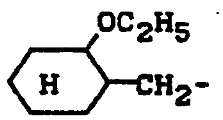 | H | $CH_3$ | 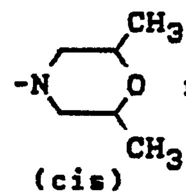 (cis) | $n_D^{20}$ : 1,4746 |

Herstellung der Ausgangsprodukte der Formel (II)

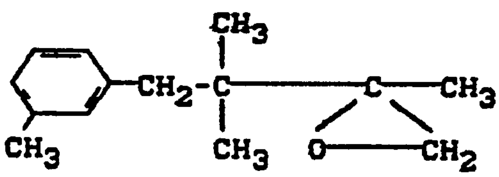

96 g (0,5 Mol) 2,2-Dimethyl-1-(3-methyl-phenyl)-butan-3-on werden in 630 ml Tetrahydrofuran gelöst. Diese Lösung wird zu einer Suspension von 132,8 g (0,6 Mol) Trimethylsulfoxoniumiodid und 68 g (0,6 Mol) Kalium-tert.-butylat in 160 ml Dimethylsulfoxid, die zuvor 6 Stunden bei 60° C gerührt wurde, getropft. Die Innentemperatur steigt auf 27° C an und man rührt 15 Stunden bei 45° C nach. Anschließend wird das Gemisch auf 2 l Eiswasser gegossen, 4 mal mit jeweils 500 ml Methylenchlorid und 2 mal mit 500 ml Wasser extrahiert. Die organische Phase wird getrocknet und das Lösungsmittel im Vakuum abdestilliert.
Man erhält 97,3 g rohes 3,3-Dimethyl-2-methyl-4-(3-methyl-phenyl)-buten-1-oxid.

Herstellung der Ausgangsprodukte der Formel (IV)

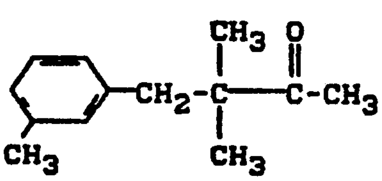

161 g (3 Mol) Kaliumhydroxid werden mit 24,4 g (0,075 Mol) Tetrabutylammoniumbromid in 1,25 l Cyclohexan vorgelegt und unter Rückfluß mit einer Lösung von 258 g (3 Mol) Me thylisopropylketon und 210 g (1,511 Mol) 3-Methylbenzylchlorid versetzt. Anschließend wird das Gemisch unter Rückfluß 30 Stunden am Wasserabscheider erhitzt, über Kieselgur abgesaugt und im Vakuum destilliert.
Man erhält so 100 g (35% der Theorie) 2,2-Dimethyl-1-(3-methyl-phenyl)-butan-3-on vom Siedepunkt Kp: 52°C/0,15 Torr.

Beispiel A

Erysiphe-Test (Gerste) / protektiv /

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20° C und einer relativen Luftfeuchtigkeit von ca. 80% aufgestellt, um die Entwicklung von Mehltaupusteln zu begünstigen.

7 Tage nach der Inokulation erfolgt die Auswertung.

Die erfindungsgemäßen Verbindungen zeigen bei diesem Test eine deutliche Überlegenheit in der Wirksamkeit gegenüber dem Stand der Technik.

## Patentansprüche

1. Substituierte Hydroxypropylamin-Derivate der allgemeinen Formel (I)

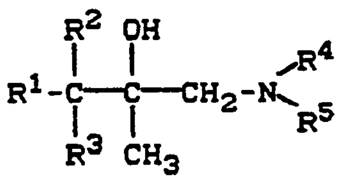

(I)

in welcher

$R^1$ für jeweils gegebenenfalls einfach oder mehrfach, gleich oder verschieden im Phenylteil durch Halogen; geradkettiges oder verzweigtes Alkyl, Alkoxy oder Alkylthio mit jeweils bis zu 5 Kohlenstoffatomen; Halogenalkyl, Halogenalkoxy oder Halogenalkylthio mit jeweils 1 oder 2 Kohlenstoffatomen und 1 bis 5 gleichen oder verschiedenen Halogenatomen substituiertes Phenyl, Phenoxy oder Phenylthio, Phenylalkyl, Phenoxyalkyl und Phenylthioalkyl mit jeweils 1 oder 2 Kohlenstoffatomen im Alkylteil, sowie für jeweils gegebenenfalls einfach oder mehrfach, gleich oder verschieden im Cyclohexylteil durch geradkettiges oder verzweigtes Alkyl und Alkoxy mit jeweils bis zu 5 Kohlenstoffatomen, Trifluormethyl oder Trifluormethoxy substituiertes Cyclohexyl, Cyclohexyloxy, Cyclohexylthio, Cyclohexylalkyl, Cyclohexyloxyalkyl und Cyclohexylthioalkyl mit jeweils 1 oder 2 Kohlenstoffatomen im Alkylteil steht,

$R^2$ für Wasserstoff oder Methyl steht;

$R^3$ für Methyl oder Ethylk steht und

$R^3$ und $R^5$ unabhängig voneinander für geradkettiges oder verzweigtes Alkyl mit 1 bis 8 Kohlenstoffatomen oder für geradkettiges oder verzweigtes Alkenyl mit 3 bis 6 Kohlenstoffatomen stehen oder

$R^4$ und $R^5$ gemeinsam mit dem Stickstoff, an das sie gebunden sind, einen gegebenenfalls einfach oder mehrfach, gleich oder verschieden durch Methyl, Ethyl oder Hydroxymethyl substituierten 5- bis 7-gliedrigen, gesättigten Heterocyclus mit 1 oder 2 Heteroatomen, stehen, sowie deren pflanzenverträgliche Süäureadditionsverbindungen und Metallsalzkomplexe, ausgenommen die Verbindung 1–N,N–Dimethylamino-2,3-dimethyl-4-(4-chlorophenyl)-2-butanol.

2. Substituierte Hydroxypropylamin-Derivate gemäß Anspruch 1, wobei die Formel (I)

$R^1$ für jeweils gegebenenfalls einfach bis dreifach, gleich oder verschieden im Phenylteil durch Fluor, Chlor, Brom, Methyl, Ethyl, Propyl, i-Propyl; n-, i-, s- und t-Butyl, n-Pentyl, 2-Methyl-2-butyl, Methoxy, Ethoxy, n- und i-Propoxy, t-Butoxy, Trifluormethyl, Trifluormethoxy oder Trifluormethylthio substituiertes Phenylmethyl, Phenoxymethyl, Phenylthiomethyl, Phenoxy oder Phenylthio steht, sowie für jeweils gegebenenfalls einfach bis dreifach gleich oder verschieden im Cyclohexylteil durch Methyl, Ethyl, n- und i-Propyl; n-, i-, s- und t-Butyl, n-Pentyl, 2-Methyl-2-butyl, Methoxy, Ethoxy, n- und i-Propoxy, t-Butoxy, Trifluormethyl oder Trifluormethoxy substituiertes Cyclohexylmethyl, Cyclohexyloxymethyl, Cyclohexylthiomethyl, Cyclohexyloxy oder Cyclohexylthio steht;

$R^2$ für Wasserstoff oder Methyl steht;

$R^3$ für Methyl oder Ethyl steht und

$R^4$ und $R^5$ unabhängig voneinander für Methyl, Ethyl, n- und i-Propyl; n-, i-, s- und t-Butyl, n-Pentyl, Allyl, 2-Butenyl oder 3-Methyl-3-butenyl stehen, oder verschieden durch Methyl, Ethyl oder Hydroxymethyl substituiertes 1-Pyrrolidinyl, 1-Piperidinyl, 1-Piperazinyl, 4-Morpholinyl oder 1-Hexahydroazepinyl stehen.

3. Verfahren zur Herstellung von substituierten Hydroxypropylamin-Derivaten der allgemeinen Formel

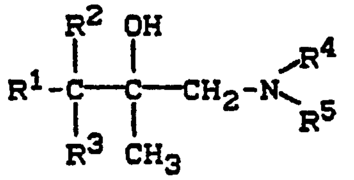

(I)

in welcher
$R^1$ für jeweils gegebenenfalls einfach oder mehrfach, gleich oder verschieden im Phenylteil durch Halogen; geradkettiges oder verzweigtes Alkyl, Alkoxy oder Alkylthio mit jeweils bis zu 5 Kohlenstoffatomen; Halogenalkyl, Halogenalkoxy oder Halogenalkylthio mit jeweils 1 oder 2 Kohlenstoffatomen und 1 bis 5 gleichen oder verschiedenen Halogenatomen substituiertes Phenyl, Phenoxy oder Phenylthio, Phenylalkyl, Phenoxyalkyl und Phenylthioalkyl mit jeweils 1 oder 2 Kohlenstoffatomen im Alkylteil, sowie für jeweils gegebenenfalls einfach oder mehrfach, gleich oder verschieden im Cyclohexylteil durch geradkettiges oder verzweigtes Alkyl und Alkoxy mit jeweils bis zu 5 Kohlenstoffatomen, Trifluormethyl oder Trifluormethoxy substituiertes Cyclohexyl, Cyclohexyloxy, Cyclohexylthio, Cyclohexylalkyl, Cyclohexyloxyalkyl und Cyclohexylthioalkyl mit jeweils 1 oder 2 Kohlenstoffatomen im Alkylteil steht,
$R^2$ für Wasserstoff oder Methyl steht;
$R^3$ für Methyl oder Ethyl steht und
$R^4$ und $R^5$ unabhängig voneinander für geradkettiges oder verzweigtes Alkyl mit 1 bis 8 Kohlenstoffatomen stehen oder
$R^4$ und $R^5$ gemeinsam mit dem Stickstoff, an das sie gebunden sind, für einen gegebenenfalls einfach oder mehrfach, gleich oder verschieden durch Methyl, Ethyl oder Hydroxymethyl substituierten 5- bis 7-gliedrigen, gesättigten Heterocyclus mit 1 oder 2 Heteroatomen, stehen,
sowie deren pflanzenverträglich Säureadditionsverbindungen und Metallsalzkomplexe, ausgenommen die Verbindung 1-N,N-Dimethylamino-,3-dimethyl-4-(4-chlorophenyl)-2-butanol, dadurch gekennzeichnet, daß man Epoxide der allgemeinen Formel (II)

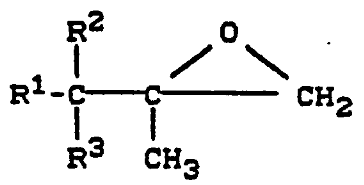

(II)

in welcher
$R^1$, $R^2$ und $R^3$ die oben angegebenen Bedeutung haben,
mit Aminen der allgemeinen Formel (III)

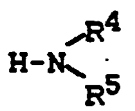

(III)

in welcher
R4 und R5 die oben angegebenen Bedeutung haben,
gegebenenfalls in Gegenwart eines Verdünnungsmittels und gegebenenfalls in Gegenwart eines Katalysators umsetzt und gegebenenfalls anschließend eine Säure oder ein Metallsalz addiert.

4. Verfahren zur Herstellung von substituierten Hydroxypropylamin-Derivaten der allgemeinen Formel (Ia)

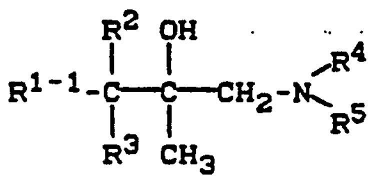

(Ia)

in welcher

$R^{1-1}$ für jeweils gegebenenfalls einfach oder mehrfach, gleich oder verschieden im Cyclohexylteil durch geradkettiges oder verzweigtes Alkyl und Alkoxy mit jeweils bis zu 5 Kohlenstoffatomen, Trifluormethyl oder Trifluormethoxy substituiertes Cyclohexyl, Cyclohexyloxy, Cyclohexylalkyl, Cyclohexyloxyalkyl mit jeweils 1 oder 2 Kohlenstoffatomen im Alkylteil steht, und
$R^2$, $R^3$, $R^4$ und $R^5$ die in Anspruch 3 angegebene Bedeutung haben,
sowie deren pflanzenverträglichen Säureadditionsverbindungen und Metallsalzkomplexen, dadurch gekennzeichnet, daß man Hydroxypropylamin-Derivate der allgemeinnen Formel (Ib)

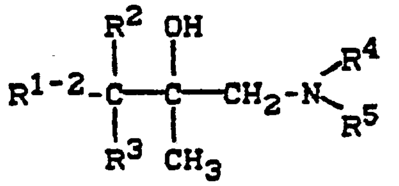

(Ib)

in welcher
$R^{1-2}$ für jeweils gegebenenfalls wie unter $R^{1-1}$ angegeben substituiertes Phenyl, Phenylalkyl, Phenoxyalkyl oder Phenoxy steht und
$R^2$, $R^3$, $R^4$ und $R^5$ die oben angegebenen Bedeutung haben,
gegebenenfalls in Gegewart eines Verdünnungsmittels und gegebenenfalls in Gegenwart eines Katalysators sowie gegebenenfalls unter erhöhtem Druck hydriert, und gegebenenfalls anschließend eine Säure oder ein Metallsalz addiert.

5. Fungizide Mittel, gekennzeichnet durch einen Gehalt an mindestens einem substituierten Hydroxypropylamin-Derivat der allgemeinen Formel (I) gemäß Anspruch 1.

6. Verwendung von substituierten Hydroxypropylamin-Derivaten der allgemeinen Formel (I) gemäß Anspruch 1 zur Bekämpfung von pilzlichen Schädlingen.

7. Verfahren zur Bekämpfung von pilzlichen Schädlingen, dadurch gekennzeichnet, daß man substituierte Hydroxypropylamin-Derivate der allgemeinen Formel (I) gemäß Anspruch 1 auf pilzliche Schädlinge und/oder deren Lebensraum einwirken läßt.

8. Verfahren zur Herstellung von fungiziden Mitteln, dadurch gekennzeichnet, daß man substituierte Hydroxypropylamin-Derivate der allgemeinen Formel (I) gemäß Anspruch 1 mit Streckmitteln und/oder oberflächenaktiven Mitteln vermischt.

## Claims

1. Substituted hydroxypropylamine derivatives of the general formula (I)

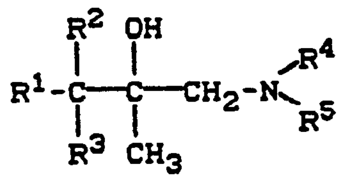

(I)

in which
$R^1$ represents phenyl, phenoxy or phenylthio, phenylalkyl, phenoxyalkyl and phenylthioalkyl, in each case having 1 or 2 carbon atoms in the alkyl part, which are in each case optionally monosubstituted or polysubstituted in the phenyl part by halogen; straight-chain or branched alkyl, alkoxy or alkylthio in each case having up to 5 carbon atoms; halogenoalkyl, halogenoalkoxy or halogenoalkylthio in each case having 1 or 2 carbon atoms and 1 to 5 identical or different halogen atoms, the substituents being identical or different, and represents cyclohexyl, cyclohexyloxy, cyclohexylthio, cyclohexylalkyl, cyclohexyloxyalkyl and cyclohexylthioalkyl, in each case having 1 or 2 carbon atoms in the alkyl part, which are in each case optionally monosubstituted or polysubstituted in the cyclohexyl part by straight-chain or branched alkyl and alkoxy in each case having up to 5 carbon atoms, trifluoromethyl or trifluoromethoxy, the substituents being identical or different,
$R^2$ represents hydrogen or methyl;
$R^3$ represents methyl or ethyl, and
$R^4$ and $R^5$, independently of one another, represent straight-chain or branched alkyl having 1 to 8 carbon atoms or straight-chain or branched alkenyl having 3 to 6 carbon atoms or
$R^4$ and $R^5$, together with the nitrogen to which they are bound, represent a 5- to 7-membered, saturated heterocyclic ring, having 1 or 2 heteroatoms, which is optionally monosubstituted or polysubstituted by methyl, ethyl or hydroxymethyl, the substituents being identical or different and their acid-addition compounds and metal salt complexes which are tolerated by plants, with the exception of the compound 1-N, N-dimethylamino-2,3-dimethyl-4-(4chlorophenyl)-2-butanol.

2. Substituted hydroxypropylamine derivatives according to Claim 1, where, in formula (I)
$R^1$ represents phenylmethyl, phenoxymethyl, phenylthiomethyl, phenoxy or phenylthio which is in each case optionally monosubstituted to trisubstituted in the phenyl part by fluorine, chlorine, bromine, methyl, ethyl, propyl, i-propyl; n-, i-, s- and t-butyl, n-pentyl, 2-methyl-2-butyl, methoxy, ethoxy, n- and i-propoxy, t-butoxy, trifluoromethyl, trifluoromethoxy or trifluoromethylthio, the substituents being identical or different, and represents cyclohexylmethyl, cyclohexyloxymethyl, cyclohexylthiomethyl, cyclohexyloxy or cyclohexylthio which is in each case optionally monosubstituted to trisubstituted in the cyclohexyl part by methyl, ethyl, n- and i-propyl; n-, i-, s- and t-butyl, n-pentyl, 2-methyl-2-butyl, methoxy, ethoxy, n- andi-propoxy, t-butoxy, trifluoromethyl or trifluoromethoxy, the substituents being identical or different;
$R^2$ represents hydrogen or methyl;
$R^3$ represents methyl or ethyl and
$R^4$ and $R^5$, independently of one another, represent methyl, ethyl, n- and i-propyl; n-, i-, s- and t-butyl, n-pentyl, allyl, 2-butenyl or 3-methyl-2-butenyl, or
$R^4$ and $R^5$, together with the nitrogen atom to which they are bound, represent l-pyrrolidinyl, l-piperidinyl, 1-piperazinyl, 4-morpholinyl or 1-hexahydroazepinyl which is in each case optionally monosubstituted to trisubstituted by methyl, ethyl or hydroxymethyl, the substituents being identical or different.

3. Process for the preparation of substituted hydroxypropylamine derivatives of the general formula

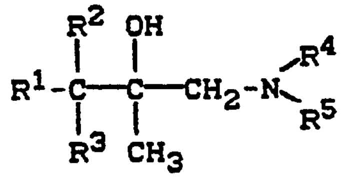

(I)

in which
$R^1$ represents phenyl, phenoxy or phenylthio, phenylalkyl, phenoxyalkyl and phenylthioalkyl, in each case having 1 or 2 carbon atoms in the alkyl part, which are in each case optionally monosubstituted or polysubstituted in the phenyl part by halogen; straight-chain or branched alkyl, alkoxy or alkylthio in each case having up to 5 carbon atoms; halogenoalkyl, halogenoalkoxy or halogenoalkylthio in each case having 1 or 2 carbon atoms and 1 to 5 identical or different halogen atoms, the substituents being identical or different, and represents cyclohexyl, cyclohexyloxy, cyclohexylthio, cyclohexylalkyl, cyclohexyloxyalkyl and cyclohexylthioalkyl, in each case having 1 or 2 carbon atoms in the alkyl part, which are in each case optionally monosubstituted or polysubstituted in the cyclohexyl part by straight-chain or branched alkyl and alkoxyin each case having up to 5 carbon atoms, trifluoromethyl or trifluoromethoxy, the substituents being identical or different,
$R^2$ represents hydrogen or methyl;
$R^3$ represents methyl or ethyl, and
$R^4$ and $R^5$, independently of one another, represent straight-chain or branched alkyl having 1 to 8 carbon atoms or straight-chain or branched alkenyl having 3 to 6 carbon atoms or
$R^4$ and $R^5$, together with the nitrogen to which they are bound, represent a 5- to 7-membered, saturated heterocyclic ring, having 1 or 2 heteroatoms, which is optionally monosubstituted or polysubstituted by methyl, ethyl or hydroxymethyl, the substituents being identical or different and their acid-addition compounds and metal salt complexes which are tolerated by plants, with the exception of the compound 1-N, N-dimethylamino-2,3-dimethyl-4-(4chlorophenyl)-2-butanol, characterized in that epoxides of the general formula (II)

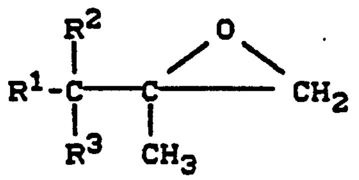

(II)

in which
$R^1$, $R^2$ and $R^3$ have the abovementioned meaning, are reacted with amines of the general formula (III)

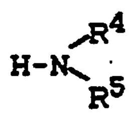

(III)

in which
$R^4$ and $R^5$ have the abovementioned meaning, if appropriate in the presence of a diluent and if appropriate in the presence of a catalyst, and, if appropriate, an acid or a metal salt is subsequently added on.

4. Process for the preparation of substituted hydroxpropylamine derivatives of the general formula (Ia)

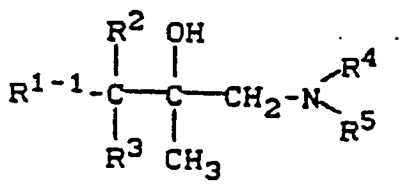

(Ia)

in which
$R^{1-1}$ represents cyclohexyl, cyclohexyloxy, cyclohexylalkyl or cyclohexyloxyalkyl each of which has 1 or 2 carbon atoms in the alkyl part and each of which is optionally monosubstituted or polysubstituted in the cycloalkyl part by straight-chain or branched alkyl or alkoxy in each case having up to 5 carbon atoms, or by trifluoromethyl or trifluoromethoxy, the substituents being identical or different, and
$R^2$, $R^3$, $R^4$ and $R^5$ have the meaning stated in Claim 3,
and their acid-addition compounds and metal salt complexes which are tolerated by plants, characterized in that hydroxypropylamine derivatives of the general formula (Ib)

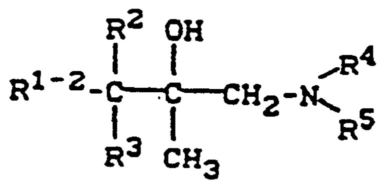

(Ib)

in which
$R^{1-2}$ represents phenyl, phenylalkyl, phenoxyalkyl or phenoxy each of which is optionally substituted as stated for $R^{1-1}$, and
$R^2$, $R^3$, $R^4$ and $R^5$ have the abovementioned meaning,
are hydrogenated, if appropriate in the presence of a diluent and if appropriate in the presence of a catalyst and if appropriate under increased pressure, and, if appropriate, an acid or a metal salt is subsequently added on.

5. Fungicides, characterized in that they contain at least one substituted hydroxypropylamine derivative of the general formula (I) according to Claim 1.

6. Use of substituted hydroxypropylamine derivates of the general formula (I) according to Claim 1 for combating fungal pests.

7. Process for combating fungal pests, characterized in that substituted hydroxypropylamine derivatives of the general formula (I) according to Claim 1 are allowed to act on fungal pests and/or on their habitat.

8. Process for the preparation of fungicides, characterized in that substituted hydroxypropylamine derivatives of the general formula (I) according to Claim 1 are mixed with extenders and/or surface active agents.

**Revendications**

1. Dérivés substitués d'hydroxypropylamine de formule générale (I)

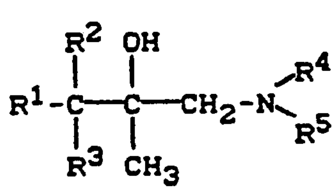

(I)

dans laquelle
$R^1$ désigne un groupe phényle, phénoxy ou phénylthio portant chacun le cas échéant dans la partie phényle un ou plusieurs substituants, identiques ou différents, halogéno; alkyle, alkoxy ou alkylthio à chaîne droite ou ramifiée ayant chacun jusqu'à 5 atomes de carbone; halogénalkyle, halogénalkoxy ou halogénalkylthio ayant chacun un ou deux atomes de carbone et 1 à 5 atomes d'halogènes identiques ou différents, un groupe phénylalkyle, un groupe phénoxyalkyle et un groupe phénylthioalkyle ayant chacun 1 ou 2 atomes de carbone dans la partie alkyle, ainsi que des groupes cyclohexyle, cyclohexyloxy, cyclohexylthio, cyclohexylalkyle, cyclohexyloxyalkyle et cyclohexylthioalkyle ayant chacun 1 ou 2 atomes de carbone dans la partie alkyle, portant éventuellement dans la partie cyclohexyle un ou plusieurs substituants, identiques ou différents, alkyle et alkoxy à chaîne droite ou ramifiée ayant chacun jusqu'à 5 atomes de carbone, trifluorométhyle ou trifluorométhoxy,
$R^2$ est l'hydrogène ou un groupe méthyle ;
$R^3$ est un groupe méthyle ou éthyle et
$R^4$ et $R^5$ représentent, indépendamment l'un de l'autre, un groupe alkyle à chaîne droite ou ramifiée ayant 1 à 8 atomes de carbone ou un groupe alcényle à chaîne droite ou ramifiée ayant 3 à 6 atomes de carbone, ou bien
$R^4$ et $R^5$ forment, conjointement avec l'atome d'azote auquel ils sont liés, un hétérocycle saturé pentagonal à heptagonal ayant 1 ou 2 hétéroatomes, portant éventuellement un ou plusieurs substituants méthyle, éthyle ou hydroxyméthyle identiques ou différents, ainsi que leurs composés d'addition d'acides et leurs complexes de sels métalliques compatibles avec les plantes, à l'exception du composé 1-N,N-diméthylamino-2, 3-diméthyl4-(4-chlorophényl)-2-butanol.

2. Dérivés substitués d'hydroxypropylamine suivant la revendication 1, dans la formule (I) desquels
$R^1$ désigne un groupe phénylméthyle, phénoxyméthyle, phénylthiométhyle, phénoxy ou phénylthio portant chacun le cas échéant, dans la partie phényle, 1 à 3 subsitutants, identiques ou différents, fluoro, chloro, bromo, méthyle, éthyle, propyle, isopropyle, n-butyle, isobutyle, sec.-butyle, tertio-butyle, n-pentyle, 2-méthyl-2-butyle, méthoxy, éthoxy, n-propoxy, isopropoxy, tertio-butoxy, trifluorométhyle, trifluorométhoxy ou trifluorométhylthio, de même qu'un groupe cyclohexylméthyle, cyclohexyloxyméthyle, cyclohexylthiométhyle, cyclohexyloxy ou cyclohexylthio portant chacun le cas échéant dans la partie cyclohexyle 1 à 3 substituants, identiques ou différents, méthyle, éthyle, n-propyle, isopropyle, n-butyle, isobutyle, sec.-butyle, tertio-butyle, n-pentyle, 2-méthyl-2-butyle, méthoxy, éthoxy, n-propoxy, isopropoxy, tertiobutoxy, trifluorométhyle ou trifluorométhoxy;
$R^2$ est l'hydrogène ou un groupe méthyle ;
$R^3$ est un groupe méthyle ou éthyle et $R^4$ et $R^5$ représentent, indépendamment l'un de l'autre, un groupe méthyle, éthyle, n-propyle, isopropyle, n-butyle, isobutyle, sec.-butyle, tertiobutyle, n-pentyle, allyle, 2-butényle ou 3-méthyl-2-butényle, ou bien $R^4$ et $R^5$ forment, conjointement avec l'atome d'azote auquel ils sont liés, un groupe 1-pipéridinyle, 1-pipérazinyle, 4-morpholinyle ou 1-hexahydroazépinyle portant chacun le cas échéant 1 à 3 substituants méthyle, éthyle ou hydroxyméthyle identiques ou différents.

3. Procédé de production de dérivés substitués d'hydroxypropylamine de formule générale

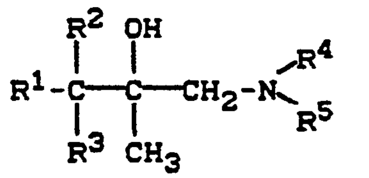

(I)

dans laquelle
$R^1$ désigne un groupe phényle, phénoxy ou phénylthio portant chacun le cas échéant dans la partie phényle un ou plusieurs substituants, identiques ou différents, halogéno; alkyle, alkoxy ou alkylthio à chaîne droite ou ramifiée ayant chacun jusqu'à 5 atomes de carbone ; halogénalkyle, halogénalkoxy ou halogénalkylthio ayant chacun un ou deux atomes de carbone et 1 à 5 atomes d'halogènes identiques ou différents, un groupe phénylalkyle, un groupe phénoxyalkyle et un groupe phénylthioalkyle ayant chacun 1 ou 2 atomes de carbone dans la partie alkyle, ainsi que des groupes cyclohexyle, cyclohexyloxy, cyclohexylthio, cyclohexylalkyle, cyclohexyloxyalkyle et cyclohexylthioalkyle ayant chacun 1 ou 2 atomes de carbone dans la partie alkyle, portant éventuellement dans la partie cyclohexyle un ou plusieurs substituants, identiques ou différents, alkyle et alkoxy à chaîne droite ou ramifiée ayant chacun jusqu'à 5 atomes de carbone, trifluorométhyle ou trifluorométhoxy,
$R^2$ est l'hydrogène ou un groupe méthyle ;
$R^3$ est un groupe méthyle ou éthyle et
$R^4$ et $R^5$ représentent, indépendamment l'un de l'autre, un groupe alkyle à chaîne droite ou ramifiée ayant 1 à 8 atomes de carbone ou un groupe alcényle à chaîne droite ou ramifiée ayant 3 à 6 atomes de carbone, ou bien
$R^4$ et $R^5$ forment, conjointement avec l'atome d'azote auquel ils sont liés, un hétérocycle saturé pentagonal à heptagonal ayant 1 ou 2 hétéroatomes, portant éventuellement un ou plusieurs substituants méthyle, éthyle ou hydroxyméthyle identiques ou différents, ainsi que de leurs composés d'addition d'acides et de leurs complexes de sels métalliques compatibles avec les plantes, à l'exception du composé 1-N,N-diméthylamino-2,3-diméthyl4-(4-chlorophényl)-2-butanol, caractérisé en ce qu'on fait réagir des époxydes de formule générale (II)

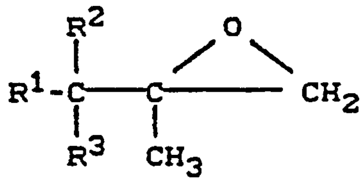

(II)

dans laquelle $R^1$, $R^2$ et $R^3$ ont la définition indiquée ci-dessus, avec des amines de formule générale (III)

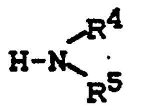

(III)

dans laquelle $R^4$ et $R^5$ ont la définition indiquée ci-dessus, le cas échéant en présence d'un diluant et en la présence éventuelle d'un catalyseur, puis on additionne éventuellement un acide ou un sel métallique.

4. Procédé de production de dérivés substitués d'hydroxypropylamine de formule générale (Ia)

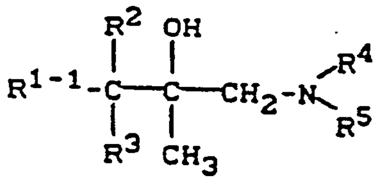

(Ia)

dans laquelle
$R^{1-1}$ représente un groupe cyclohexyle, cyclohexyloxy, cyclohexylalkyle ou cyclohexyloxyalkyle ayant chacun un ou deux atomes de carbone dans la partie alkyle et chacun portant le cas échéant dans la partie cyclohexyle un ou plusieurs substituants, identiques ou différents, alkyle et alkoxy à chaîne droite ou ramifiée ayant chacun jusqu'à 5 atomes de carbone, trifluorométhyle ou trifluorométhoxy et
$R^2$, $R^3$, $R^4$ et $R^5$ ont la définition indiquée dans la revendication 3, ainsi que de leurs composés d'addition d'acides et leurs complexes de sels métalliques compatibles avec les plantes, caractérisé en ce qu'on hydrogène des dérivés d'hydroxypropylamine de formule générale (Ib)

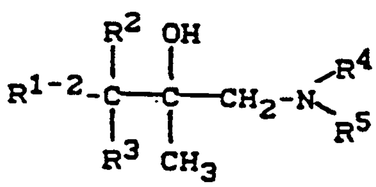

(Ib)

dans laquelle
$R^{1-2}$ représente un groupe phényle, phénylalkyle, phénoxyalkyle ou phénoxy, chacun étant substitué le cas échéant comme indiqué pour $R^{1-1}$ et
$R^2$, $R^3$, $R^4$ et $R^5$ ont la définition indiquée ci-dessus, le cas échéant en présence d'un diluant et en la présence éventuelle d'un catalyseur, ainsi que le cas échéant sous pression élevée, puis on additionne éventuellement un acide ou un sel métallique.

5. Compositions fongicides, caractérisées par une teneur en au moins un dérivé substitué d'hydroxypropylamine de formule générale (I) suivant la revendication 1.

6. Utilisation de dérivés substitués d'hydroxypropylamine de formule générale (I) suivant la revendication 1 pour combattre des champignons parasites.

7. Procédé pour combattre des champignons parasites, caractérisé en ce qu'on fait agir des dérivés substitués d'hydroxypropylamine de formule générale (I) suivant la revendication 1 sur les champignons parasites et/ou sur leur milieu.

8. Procédé de préparation de compositions fongicides, caractérisé en ce qu'on mélange des dérivés substitués d'hydroxypropylamine de formule générale (I) suivant la revendication 1 avec des diluants et/ou des agents tensio-actifs.